# EUROPÄISCHE PATENTSCHRIFT

(11) **EP 1 328 289 B1**
(45) Veröffentlichungstag und Bekanntmachung des Hinweises auf die Patenterteilung: **03.09.2008**
(21) Anmeldenummer: 01983562.8
(22) Anmeldetag: 19.10.2001
(51) Int. Cl.: A61K 38/39, A61K 39/395, A61P 7/00, G01N 33/50

(54) **OXIDIERTE PROTEINE UND DEREN BIOLOGISCHE UND THERAPEUTISCHE AKTIVITÄT SOWIE DIAGNOSTISCHE VERWENDUNG ÜBER DIE HEMMUNG DER INTERAKTION ZWISCHEN OXIDIERTEN PROTEINEN UND CD36**
OXIDIZED PROTEINS, THEIR BIOLOGICAL ACTIVITY, AND THERAPEUTIC AND DIAGNOSTIC MEASURES, WHICH ARE DERIVED FROM THE ACTIVE MECHANISM, FROM THE USE OF THESE PROTEINS OR FROM THE INHIBITION THEREOF
PROTEINES OXYDEES, LEUR EFFET BIOLOGIQUE ET MESURES THERAPEUTIQUES ET DIAGNOSTIQUES DECOULANT DU MECANISME D'ACTION, DE L'UTILISATION DE CES PROTEINES OU DE LEUR INHIBITION

(30) Priorität: 20.10.2000 DE 10051983; 02.10.2001 DE 10148624
(43) Veröffentlichungstag der Anmeldung: 23.07.2003
(60) Teilanmeldung: 08012302.9
(73) Patentinhaber: Hamburger Stiftung zur Förderung von Wissenschaft und Kultur, 20354 Hamburg (DE)
(72) Erfinder: KEHREL, Beate, 48161 Münster (DE); BRODDE, Martin, 48161 Münster (DE)
(74) Vertreter: Weickmann, Franz Albert
(86) Internationale Anmeldenummer: PCT/EP2001/012129
(87) Internationale Veröffentlichungsnummer: WO 2002/032445

(56) Entgegenhaltungen:
- WO-A-00/28072
- WO-A-91/10684
- DAWSON DAVID W ET AL: "CD36 mediates the in vitro inhibitory effects of thrombospondin-1 on endothelial cells." JOURNAL OF CELL BIOLOGY, Bd. 138, Nr. 3, 1997, Seiten 707-717, XP002215771 ISSN: 0021-9525
- PIETSCH ANGELIKA ET AL: "Lovastatin reduces expression of the combined adhesion and scavenger receptor CD36 in human monocytic cells." BIOCHEMICAL PHARMACOLOGY, Bd. 52, Nr. 3, 1996, Seiten 433-439, XP002215770 ISSN: 0006-2952
- NGUYEN-KHOA THAO ET AL: "Oxidized low-density lipoprotein induces macrophage respiratory burst via its protein moiety: A novel pathway in atherogenesis?" BIOCHEMICAL AND BIOPHYSICAL RESEARCH COMMUNICATIONS, Bd. 263, Nr. 3, 5. Oktober 1999 (1999-10-05), Seiten 804-809, XP002215772 ISSN: 0006-291X in der Anmeldung erwähnt
- MORAGA FABIAN ET AL: "Activation of primary human monocytes by the oxidized form of alpha1-antitrypsin." JOURNAL OF BIOLOGICAL CHEMISTRY, Bd. 275, Nr. 11, 17. März 2000 (2000-03-17), Seiten 7693-7700, XP000961184 ISSN: 0021-9258
- CROMBIE RENE ET AL: "Identification of a CD36-related thrombospondin 1-binding domain in HIV-1 envelope glycoprotein gp120: Relationship to HIV-1-specific inhibitory factors in human saliva." JOURNAL OF EXPERIMENTAL MEDICINE, Bd. 187, Nr. 1, 5. Januar 1998 (1998-01-05), Seiten 25-35, XP002215774 ISSN: 0022-1007 in der Anmeldung erwähnt
- LUPIDI G ET AL: "Peroxynitrite-mediated oxidation of fibrinogen inhibits clot formation" FEBS LETTERS, ELSEVIER SCIENCE PUBLISHERS, AMSTERDAM, NL, Bd. 462, Nr. 3, 3. Dezember 1999 (1999-12-03), Seiten 236-240, XP004260622 ISSN: 0014-5793

## Beschreibung

Die vorliegende Erfindung betrifft Substanzen gemäß den Ansprüchen, die die Bindung von oxidierten Proteinen an CD36 oder die durch Interaktion von CD36 mit oxidierten Proteinen induzierten Funktionen von CD36 hemmen und ihre Verwendung als Arzneimittel für Menschen und Tiere.

Die oxidative Modifikation von Proteinen wird als kritischer Schritt für die Pathogenese von verschiedenen Erkrankungen, die von Atherosklerose (Arteriosklerose), über neurodegenerative Erkrankungen bis hin zum Alterungsprozeß selbst reichen, angesehen (Holvoet and Collen, 1997 Curr Opin Lipidol, Witztum and Steinberg 1991, J Clin Invest 88, 1785-92, Smith MA et al., 1996, Nature, Oxidative damage in Alzheimers, Stadtmann ER, Protein Oxidation and Aging, 1992, Science 257: 1220-24). Hohe LDL-Konzentrationen im Blut gelten als Hauptrisikofaktor für die Entwicklung von arteriosklerotischen (atherosklerotischen) Gefäßerkrankungen (Brown MS, Goldstein IL, 1986, Science 232: 34-37).

Jedoch besteht heute überwältigende Evidenz zu glauben, dass nicht LDL selber, sondern seine oxidierte Form der entscheidende Trigger für die krankheitserregenden Veränderungen, die zur Arteriosklerose führen, ist (Steinberg D, Circulation 95: 1062-71, 1997). Das US Patent No 5,756,067 beschreibt, daß die Messung von Cholesterin, Triglyzeriden und Lipoproteinen als Risikomarker für eine sich entwickelnde Atherosklerose noch nicht ausreichend ist, da etwa die Hälfte aller Herzerkrankungen aufgrund von Atherosklerose in Patienten vorkommt, welche normale Plasma Triglyzerid- und normale Cholesterinwerte haben und weil der angiographische Nachweis von Atherosklerose auch in Patienten mit normalen Lipidwerten geführt werden kann. Weitere noch nicht publizierte Vorgänge müssen also an der Entstehung der Arteriosklerose ursächlich beteiligt sein.

Die Oxidation der Lipide im LDL, entweder in vitro z.B. durch Kupfer-induzierte Oxidation, oder in vivo, führt zur Bildung von reaktiven Aldehyden (WO 98/59248). Die Aufnahme von oxidiertem LDL (oxLDL) durch Makrophagen führt zur Bildung von sogenannten Schaumzellen, ein Vorgang, welcher als initiierender Schritt bei der Entwicklung der Arteriosklerose angesehen wird (WO 98/59248).

Als für diesen Prozeß verantwortlich wird die Oxidation des Lipidanteils im LDL angesehen. Daher wird oxLDL für Untersuchungen zur Entstehung der. Arteriosklerose von fast allen Wissenschaftlern durch CuSO₄-Zusatz oder mit Malondialdehyd, einem Endprodukt der Lipidperoxidation, erzeugt. Die Konzentration an oxLDL im Plasma von Patienten mit koronarer, primärer Herzkrankheit oder Transplantations-assoziierter koronarer Herzkrankheit korreliert stark mit dem Fortschreiten der Erkrankung, während in gesunden Kontrollpersonen keine erhöhten oxLDL-Spiegel gemessen werden können (Holvoet, et al, Circulation 98: 1487-94, 1998). Auch chronische Nierenerkrankungen und Transplantatabstoßungen sind mit hohen oxLDL-Spiegeln assoziiert (Holvoet, Collen Thromb Haemost 76(5): 663-9, 1996 + ATVB 18(1) 100-7, 1998).

Auch die Oxidation des Proteinanteils im LDL kann physiologische/pathophysiologische Veränderungen hervorrufen. So führte delipidiertes HOCl-oxLDL zur Induktion des oxidativen Burst in Makrophagen (Nguyen-Khoa et al., BBRC 263: 804-9, 1999) und HOCl-oxLDL rief Thrombozytenaggregation hervor (Volf et al, ATVB 20(8): 2011-18, 2000).

Reaktive Oxidantien werden vom Körper durch Phagocyten freigesetzt und spielen eine bedeutende Rolle bei der Abwehr gegen Krankheitserreger, bei der Tumorüberwachung und bei allen Entzündungsprozessen (Babior, NEJMed 298: 659-663, 1978, Weiss J, NEJMed 320: 365-376, 1989). Neben professionellen" Phagocyten, wie Granulozyten und Monozyten, können auch andere Zellen, wie z.B. Endothelzellen oder glatte Muskelzellen, reaktive Oxidantien produzieren und abgeben.

Zu diesen oxidierenden Substanzen gehören O₂, Superoxid, Hydrogenperoxid, Peroxynitrite, OH-Radikate, hypochlorige Säure HOCI, Cl₂-Gas und Chloramine. Welche Prozesse im Detail an der Entstehung dieser oxidativen Substanzen beteiligt sind, ist noch unklar. Ceruloplasmin, 15-Lipoxygenase, Myeloperoxidase (MPO) und Nitric oxid-Synthase (NO-S) wurden in arteriosklerotischen Läsionen im Tier und Mensch gefunden und könnten zur Oxidierung von LDL beitragen (Carr et al., ATVB 20:1716-23, 2000). Ein möglicher Oxidierungsweg involviert die MPO. MPO, ein Häm-Protein Enzym, kann halogenieren und peroxidieren (Carr et al.). Das am besten beschriebene Produkt der Myeloperoxidase ist hypochlorige Säure HOCl⁻, Cl⁻ + H₂O₂ + H⁺ → HOCl + H₂O. Hypochlorit modifizierte Proteine, insbesondere HOCl-oxLDL, werden in arteriosklerotischen Läsionen gefunden (Hazell et al 1996). HOCI-Modifikation von Proteinen spielt auch eine Rolle bei anderen Erkrankungen, wie z.B. entzündlichen Gelenkerkrankungen (Davies et al., Free-Radical-Biol-Med 15(6): 637-43, 1993), Gerinnungsstörungen (Oxidation von Thrombomodulin) (Glaser et al., J Clin Invest 90(6): 2565-73, 1992), Gewebszerstörung durch Granulozyten bei Entzündungsreaktionen allgemein (Schraufstatter et al., J Clin Invest 85(2): 554-62, 1990), Ischämie, Reperfusionsschaden (Samanta et al., Free Radic Res Commun 7(2): 73-82, 1989), Glomerulonephritis (Shah et al., J Clin Invest 79(1): 25-31, 1987) und Immunregulation (NK-cell-Apoptose) (Hansson et al., J Immunol 156(1): 42-7, 1996).

CD36, auch Glykoprotein IIIb (GPIIIb) oder Glykoprotein IV (GP IV) genannt, ist ein Hauptglykoprotein der Plättchen, Endothelzellen, Monozyten, Erythroblasten, Epithelzellen und einiger Tumorzelllinien, wie Melanomzellen und Osteosarkomzellen (Asch et al J. Clin. Invest. 79:1054-1061 (1987), Knowles et al J. Immunol. 132, 2170-2173 (1984), Kieffer et al Biochem. J. 262:835-842 (1989)). Es gehört zur Familie der Klasse B scavenger" Rezeptoren. Mitglieder der Familie sind das integrale lysosomale Membranprotein LIMP-II (lysosomal integral membrane protein II, Vega et al 1991) das CLA-1 (CD36 and LIMP-II analogous, Calvo und Vega 1993), das FAT-Protein der Adipozytenmembran (Abumrad et al 1993), PAS IV aus Brustepithelzellen (Greenwalt et al 1990 und 1992) und das SR-B1 (Acton et al 1994).

Das FAT-Protein der Adipozyten ist an der Bindung und dem Transport langkettiger Fettsäuren beteiligt. Das PAS IV-Protein ist ein integrales Membranprotein laktierender Brustepithelzellen und wird im apikalen Plasmalemma konzentriert. Mit der Sekretion von Triglyceriden gelangt es in die Milch und wird in der MFGM (milk-fat globule membrane)-Fraktion gefunden. Die Sequenz von PAS IV ist fast identisch mit CD36, es liegen aber Unterschiede in der Glykosilierung vor.

SR-B1 ist ein "scavenger receptor" für LDL (Acton et al 1994). CD36 besteht aus einer einzigen stark glykosilierten Polypeptidkette mit einem aparenten Molekulargewicht von 88000 im reduzierten und nicht reduzierten Zustand und einem isoelektrischen Bereich zwischen 4,4 und 6,3 (McGregor et al 1980, Clemetson 1987). Daß sich kein klar definierter isoelektrischer Punkt bestimmen lässt, liegt am variablen Gehalt an Sialinsäure (McGregor et al 1981). Der Kohlenhydratanteil von 26% und eine starke Hydrophobie gibt dem CD36 eine hohe Resistenz gegenüber Abbau durch Proteasen, solange das Protein sich in der Membran befindet (Greenwalt et al 1992). Dies bewirkt, dass das Protein in Regionen, an denen Entzündungsprozesse stattfinden, vor "Angriffen" geschützt ist. CD36 hat N- und O-glykosidische Bindungen. Die aus plazentarer cDNA für CD36 abgeleitete Aminosäuresequenz (Oquendo et al 1989) zeigt mehrere hydrophobe Bereiche und vermutlich zwei transmembranäre Bereiche.

Einige Funktionen sind für CD36 postuliert worden. So wurde es als Rezeptor für Kollagen beschrieben (Tandon et al 1989). Gereinigtes CD36 bindet an Fibrillen von Kollagen Typ I, und Fab-Fragmente eines polyklonalen Antikörpers gegen CD36 hemmen die Kollagen-induzierte Aggregation.

Analysen von Plättchen, denen das CD36 fehlt, zeigen aber, dass CD36 für die Aktivierung von Plättchen mit Kollagen nicht uhbedingt notwendig ist.

Unsere gemeinsamen Untersuchungen mit Kollegen der Arbeitsgruppe von J.J. Sixma (Utrecht) zeigten keine Unterschiede in der Adhäsion von CD36-defizienten Plättchen und Kontrollplättchen an bovine oder humane Kollagene Typ I oder III im statischen System und unter Einfluß von geringen, mittleren und hohen Scherraten in der Perfusionskammer bei Verwendung von Heparinblut und bei physiologischen Ca²⁺-Konzentrationen (Saelman et al 1994).

CD36 "defiziente Plättchen aggregieren normal mit Horm-Kollagen, einem Gemisch aus equinem Typ I und III Kollagen, und mit gereinigtem bovinen und humanen Kollagen Typ I und III (Kehrel et al 1991 und 1993). Die Sekretion der á-Granula und dichten Granula induziert durch Typ oder III Kollagen unterscheidet sich nicht bei CD36-defizienten Plättchen und Kontrollplättchen (Kehrel et al 1993). Daniel und Mitarbeiter zeigten, daß die Signalübertragung nach Aktivierung mit Kollagen Typ I in CD36-defizienten Plättchen und Kontrollplättchen gleich verläuft (Daniel et al 1993).

CD36 ist ein Rezeptor für Thrombospondin-1 (TSP-1) (Asch et al 1987, McGregor et al 1989). Auf ruhenden Thrombozyten ist CD36 Threonin(92) phosphoryliert. Dephosphorylierung ermöglicht das Anbinden von Thrombospondin (Asch, Science 1993). Gereinigtes CD36 bindet spezifisch an Thrombospondin. Diese Bindung ist Ca²⁺-abhängig und kann durch RGD-Peptide nicht gehemmt werden. Der monoklonale Antikörper OKM5, der gegen CD36 gerichtet ist, hemmt die Bindung von Thrombospondin an mit Thrombin aktivierte Plättchen (Asch et al 1989). Leung und Mitarbeiter beschrieben, daß auf dem CD36 zwei Peptidbereiche die Bindung von Thrombospondin beeinflussen. Peptid 139-155 verstärkt die Plättchenaggregation im Plättchen-reichen Plasma, die durch ADP oder Kollagen induziert wurde. Peptid 93-110 hingegen hemmt die Kollagen-induzierte Aggregation teilweise und blockiert auch die Bindung von CD36 an immobilisiertes Thrombospondin. Dieses Peptid kann das Thrombospondin allein nicht binden, aber in Gegenwart des Peptids 139-155 (Leung et al 1992, Pearce et al 1993). Die Sequenz SVTCG des Thrombospondins bindet mit hoher Affinität an CD36 (Li et al 1993). Silverstein et al (1992) belegten durch Experimente mit "sense" und "antisense" transfizierten Melanomzellen die Bedeutung von CD36 für die Thrombospondinbindung. Die Bindungsstelle auf CD36 für Thrombospondin liegt zwischen Aminosäure 93-120 (Frieda et al 1995).

CD36 wird auch als Bindungsvermittler zwischen Plättchen, Endothelzellen, Monozyten oder C32-Melanomzellen einerseits und mit dem Malariaparasiten *Plasmodium falciparum* befallenen Erythrozyten andererseits, beschrieben (Barnwell et al 1989). Die Bindung infizierter Erythrozyten an Kapillarendothelzellen, Sequestration genannt, ist von entscheidender Bedeutung für den oft tödlichen Ausgang der Malaria tropica, wenn die Sequestration im Gehirn stattfindet (zelebrale Malaria).

Infizierte Erythrozyten binden an immobilisiertes, gereinigtes CD36 (Ockenhouse et al 1989). COS-Zellen, in denen die cDNA für CD36 exprimiert wurde, erlangen die Fähigkeit, Malaria-infizierte Erythrozyten zu binden (Oquendo et al 1989). Mit Hilfe von anti-Idiotyp-Antikörpern gegen den monoklonalen anti CD36-Antikörper OKM5 wurde ein Bindungspartner auf infizierten Erythrozyten, das Sequestrin, gefunden (Ockenhouse et al 1991). Durch den Kontakt mit infizierten Erythrozyten werden Plättchen und Monozyten aktiviert (Ockenhouse et al 1989). CD36-defiziente Plättchen zeigten in den Händen von Tandon et al (1991) keine Bindungsfähigkeit für infizierte Erythrozyten. Wir beobachteten dagegen, daß die Bindung nur in Gegenwart von EDTA gestört ist. In Gegenwart von Ca²⁺ und Mg²⁺ konnten wir klar Rosetting von *Plasmodium falciparum*-infizierten Erythrozyten und CD36-defizienten Plättchen beobachten (Kronenberg et al 1992). Es sind neben CD36 noch andere Bindungsvermittler für Malaria-infizierte Erythrozyten beschrieben worden, darunter Thrombospondin, welches für diese Funktion zweiwertige Kationen benötigt (Berendt et al 1989, Roberts et al 1985). Thrombospondin könnte die von uns beobachtete Bindung von CD36 "defizienten Plättchen an infizierte Erythrozyten vermitteln. Die Bindung von Plasmodium falciparum-infizierten Erythrozyten an CD36 wird durch die CD36 Peptide 145-171 und 156-184 inhibiert (Baruch et al 1999). Auch eine Rolle des CD36 bei der Signalübertragung wird häufig diskutiert (Shattil und Brugge 1991). Bei der Immunpräzipitation von CD36 aus ruhenden Plättchen werden die Tyrosinkinasen der src-Genfamilie pp60^{fyn}, pp62yes und pp54/58^{lyn} mitpräzipitiert, was für eine enge Assoziation mit dem CD36 spricht (Huang et al 1991). Die Bedeutung dieser Entdeckung ist noch unklar. Einige Antikörper gegen CD36 aktivieren Plättchen und Monozyten (Aiken et al 1990, Schüepp et al 1991). Der lgM-Antikörper NL07 aktiviert Plättchen mit Hilfe des Komplementsystems (Alessio et al 1993). Als weitere Funktion von CD36 wird eine Rolle bei der thrombotisch thrombozytopenischen Purpura (TTP) beschrieben (Lian et al 1991). Ein Protein (p37), welches im Plasma von TTP-Patienten vorkommt, agglutiniert Plättchen, vermittelt über CD36. Die Bedeutung dieses Befundes ist noch unbekannt. Das Peptid VTCG aus dem Thrombospondin hemmt die Phosphatidylinositol (3.4) biphosphat-Synthese in mit Thrombin aktivierten Plättchen. CD36 ist einer der Thrombospondin-Rezeptoren, welche eine späte Aktivierung der Pl-3-Kinase und der Phospholipase C vermitteln (Trumel, Payrastre, Mazarguil, Chap, Plantavid, persönliche Mitteilung).

CD36 ist beteiligt am Transport von langkettigen Fettsäuren in Muskelgewebszellen.

Eine Überexpression von CD36 in Muskelzellen von transgenen Mäusen führte zu verstärkter zellulärer Aufnahme von Fettsäuren, verstärkte die Fettsäureoxidation durch kontraktile Muskeln und reduzierte die Konzentration von Triglyceriden und freien Fettsäuren im Plasma. Die Mäuse hatten ein geringeres Körpergewicht, insbesondere geringeres Körperfett, als ihre Kontrollverwandten.

Fehlen von CD36 beim Menschen führt zum Verlust der Aufnahme von langkettigen Fettsäuren, die Hauptenergiequelle für den Herzmuskel, in die Herzmuskelzellen und konsequent zu erhöhtem Auftreten von angeborenen hypertrophen Kardiomyopathien (Fuse et al 1998). Auch CD36 defiziente Mäuse zeigen einen defekten Transport von Fettsäuren in die Zellen und einen gestörten Lipoproteinstoffwechsel -(Febbraio et al 1999).

CD36 vermittelt die Arachidonsäure vermittelte Plättchenaggregation (Dutta-Roy et al 1996) und bindet an negativ geladene Phospholipide in Zellmembranen (Ryeom et al 1996). Insbesondere Phosphatidylserine (PS) und Phosphatidylinositol (Pl) werden von CD36 spezifisch mit hoher Affinität gebunden (Rigotti et al 1995). Da apoptotische Zellen Phosphatidylserine an ihrer Oberfläche exponieren, kann über CD36 ein Kontakt zu phagozytotischen Zellen vermittelt werden (Fadok et al 1998, Alberts et al 1998). Phosphatidylserin bindet vermutlich an die CD36-Sequenz 162-183 (Yamaguchi et al 2000). CD36 bindet an Cholesteryl-hemisuccinat und kann über diese Reaktion leicht gereinigt werden (Kronenberg et al 1998).

Die Hauptfunktion von CD36 ist wohl seine Rolle als Rezeptor für oxidiertes LDL. Diese Rolle wurde von Endemann et al 1993 zuerst beschrieben. Transfektionsexperimente mit einem cDNA-Klon, der für CD36 codiert, in der humanen Makrophagen ähnlichen THP-Zelllinie führten zu einer neu erlangten Bindungsfähigkeit der Zelle für Cu²⁺-oxidiertes LDL. Der monoklonale CD36-spezifische Antikörper OKM5 hemmt die Bindung von Cu²⁺-oxLDL an Plättchen um 54%. Die Bindungsstelle für Cu²⁺-oxLDL liegt im Bereich der CD36 Sequenz 155-183 (Puente et al 1996). Nicholson et al beschrieben, dass Cu²⁺-oxLDL vermutlich über seinen Lipidanteil an CD36 bindet (Nicholson et al 1995). Makrophagen von Blutspendern, denen das CD36 auf den Monozyten fehlt, haben im Vergleich zu Kontrollen eine deutlich reduzierte (-40%) Aufnahme von oxLDL (Nozaki et al 1995).

Vitamin E (alpha-Tocopherol) hemmt die Aufnahme von Cu²⁺-oxLDL in glatte Muskelzellen der Aorta, indem es CD36 inhibiert (Ricciarelli et al 2000). Die Bindung von oxLDL an Maus CD36 wird teilweise durch oxidierte Phospholipide- übernommen, die mit Lipid und Proteinanteil assoziiert sind (Boullier et al 2000). Kürzlich wurde festgestellt, daß CD36 nicht nur der Rezeptor für Cu²⁺-oxLDL ist, sondern auch für NO₂-LDL und daß CD36 für die NO₂-LDL mediierte Schaumzellbildung verantwortlich ist (Podrez et al 2000).

Diese Bindung wird durch Lipid-Oxidationsprodukte von 1-Palmitoyl-2-arachidonyl-sn-glycero-3-phosphocholine kompetitiv inhibiert. Die Autoren spekulieren daher, dass die Myeloperoxidase katalysierte Peroxidation von Lipiden dazu dient, phospholipidhaltige Angriffsziele für die Phagozytose durch CD36-haltige Zellen zu markieren. Im Einklang mit seiner Rolle als Rezeptor für oxLDL wird CD36 auf mit Lipiden beladenen Makrophagen in arteriosklerotischen Plaques gefunden (Nakata et al 1999) und glatte Muskelzellen können durch Expression von CD36 in vivo zu Schaumzellen werden (Ohya et al 2000).

Fehlen von CD36 scheint ein Schutz gegen Arteriosklerose zu sein. So entwickeln Mäuse, denen das ApoE-Protein fehlt, bei entsprechender Diät arteriosklerotische Plaques. Wird diesen Mäusen zusätzlich das CD36 Gen ausgeknockt, so entwickeln die Mäuse unter gleichen Bedingungen wie ihre CD36 haltigen Kontrollverwandten 76% weniger arteriosklerotische Läsionen im Aortenbaum unter fettreicher Diät und 45% weniger Plaques im Aortensinus bei normaler Diät.

Makrophagen von CD36 und ApoE doppel knock-out Mäusen internalisierten mehr als 60% weniger Kupfer-oxidiertes LDL und NO₂-LDL (Febbraio et al 2000).

Eine Blockade der thrombotischen und arteriosklerotischen Funktionen von CD36 bei gleichzeitiger Nichtbeeinflussung der wichtigen CD36 vermittelten Aufnahme von langkettigen Fettsäuren in die Zelle wäre ein wichtiger Schritt vorwärts zur Bekämpfung von Gefäßerkrankungen.

Diese Aufgabe wurde durch die vorliegende Erfindung gelöst.
Im Rahmen der vorliegenden Erfindung wurde festgestellt, dass Zellfunktionen, die oxLDL über CD36 auslöst, auch von anderen Substanzen ausgelöst werden können. Überraschenderweise handelt es sich bei diesen anderen Substanzen um oxidierte Proteine, die keinen Lipidanteil haben müssen. Im Körper werden Proteine bei der Infektabwehr, bzw. in arteriosklerotischen Plaques, oder bei akuten und chronischen Entzündungen oxidiert.

Im folgenden seien beispielhaft einige Reaktionen aufgeführt, die nicht nur von oxLDL, sondern auch von anderen oxidierten Proteinen über CD36 ausgelöst werden:
(1) Aktivierung von Thrombozyten
   → einerseits Thrombosen, Herzinfarkt, Schlaganfall, aber andererseits auch Blutstillung
(2) Schädigung von Endothelzellen
   → Ischämie, Entzündungsreaktionen, Ödembildung, Störung der Prostacyclin-Freisetzung
(3) Aktivierung von Leukozyten, z.B.
   a) Erhöhte Adhäsion von Leukozyten an Endothelzellen
   b) Förderung der Transmigration von Leukozyten durch Endothel und Epithelien
   c) "Homing" von Leukozyten in arteriosklerotische Plaques
   d) "Priming" und Auslösen des oxidativen Burst in Phagozyten
   e) Tissue Faktor Expression auf Monozyten, insbesondere Verstärkung der durch Lipopolysaccharid (LPS) ausgelösten Reaktion
      → Schäden durch Entzündungsreaktion, Thrombosen, etc.
(4) Aktivierung von glatten Muskelzellen (SMC's)
   a) Proliferation von SMC's
   b) Intimaverdickung in arteriosklerotischen Plaques
      → Reocclusion nach Bypass, Stent, PTCA; Fortschreiten von Arteriosklerose
(5) Stimulation der Renin-Freisetzung aus juxta-glomerulären Zellen
   → Renin-abhängiger Bluthochdruck bei Nierenerkrankungen
(6) Bildung von Schaumzellen über Stimulation der Aufnahme von co-inkubiertem LDL durch Makropinozytose
   → Entwicklung/Verstärkung von Arteriosklerose
(7) Apoptose von Gefäßzellen im Zentrum von arteriosklerotischen Läsionen
   → Nekrose, Plaque-Ruptur
(8) Stimulierung der Expression von Matrix-Metalloproteinasen in Endothelzellen
   → Stimulation der Ruptur von arteriosklerotischen Plaques.

Daneben wurde in dieser Erfindung festgestellt, dass nicht nur IDAAT (immune defense activated antithrombin, Patentanmeldung Nr. DE 100 45 047.4) an HIV GP120 und an CD4 bindet, sondern auch andere oxidierte Proteine. HIV GP120 enthält eine CD36 homologe Sequenz (Crombie et al 1998).

Es wurde in dieser Erfindung auch festgestellt, dass nicht nur IDAAT, sondern auch andere oxidierte Proteine die Bindung von Thrombospondin an Zellen wie Thrombozyten, Monozyten, Endothelzellen und T-Zellen bewirken. Es ist daher zwingend anzunehmen, dass oxidierte Proteine allgemein Thrombospondin vermittelte Zellreaktionen induzieren, wie die Inhibierung der Angiogenese, die Abwehr von HIV-Infektionen, die Regulation von Entzündungsprozessen durch z.B. "Downregulation" von IL-12 in Monozyten und "Upregulation" von IL-10. Damit können auch oxidierte Proteine selber bei bestimmten Krankheitsprozessen therapeutisch nutzbare Wirkung haben.

Des weiteren wurde im Rahmen dieser Erfindung festgestellt, dass die durch oxidierte Proteine hervorgerufenen pathologischen Zellfunktionen durch Substanzen gemäß den Ansprüchen, die die Interaktion zwischen CD36 und oxidierten Proteinen hemmen oder die mit an CD36 gebundenem TSP konkurrieren können, inhibiert werden können.

Als Beispiele für solche Substanzen sei hier lösliches Thombospondin-1 und die von uns hergestellten monoklonalen anti CD36 Antikörper Klon 37, 13 und 7 beschrieben.

### Beispiele:

### 1. Isolierung von humanem Thrombospondin-1

Die Isolierung von humanem Thrombospondin-1 aus Thrombozyten erfolgte wie in Kehrel et al 1991 beschrieben. Auf die Beschreibung von Kehrel et al. 1991, wird hiermit Bezug genommen. Abweichend davon wurden die Plättchen aber mit Thrombin aktiviert und EDTA im Waschpuffer durch Na-Citrat (0.08 M) ersetzt. Zusätzlich wurde dem Aggregationspuffer und allen Puffern für die nachfolgenden Reinigungsschritte Ca²⁺ in einer Konzentration von 2 mM zugesetzt.

### 2. Hybridomkultur zur Herstellung von monoklonalen Antikörpern

Die monoklonalen Antikörper gegen CD36 wurden weitgehend nach den Anleitungen von Peters und Baumgartner (1990) durchgeführt.

8-12 Wochen alte Balb/c Mausweibchen wurden mit gereinigtem CD36 (50 µg/Boost) immunisiert. Zur Immunisierung wurde das lange Immunisierungsschema (4 Monate) nach Baumgartner et al 1990 benutzt. Etwa 14 Tage vor dem geplanten Fusionstermin wurde den Tieren Blut entnommen und der lgM und lgG-Titer gegen CD36 im Serum der Mäuse bestimmt. Unterschied sich der lgG-Titer noch bei einer Verdünnung von 1:100.000 deutlich von der Kontrolle, so wurden die Milzzellen mit Ag 8.653 Zellen fusioniert. Die Ag 8.653 Zellen wurden mit 0.13 M 8-Azaguanin im Kulturmedium selektioniert. Lymphozyten aus der Milz wurden präpariert und mit Ag 8.653 fusioniert. Fusionierte Zellen (1×10⁶ Milzzellen/ml) wurden direkt nach der Fusion für 24 Stunden in Selektionsmedium (Kulturmedium mit Zusatz von Hypoxanthin, 27.2 µg/ml und Azaserin (50 µg/ml)) in einer Zellkulturflasche (75 ml) inkubiert. Dabei haften die Makrophagen aus der Milz an die Kunststoffoberfläche und stören nicht mehr die eigentliche Kultur der Hybridome in einer 96-Well-Platte.

Die Klonierungsschritte wurden durch das Verdünnungsverfahren ("limited-dilution-cloning") mit einer Aussaatwahrscheinlichkeit von 0.5 Zellen pro Loch der 96-Well-Platte nach Würzner 1990 vorgenommen. Überstände von Hybridomen wurden im ELlSA-Verfahren auf die Produktion von lgG, bzw. lgM getestet. lgG-positive Zellkulturüberstände wurden mit mehreren Verfahren auf ihre Spezifität gegen CD36 getestet:
19 für CD36 spezifische Klone wurden erhalten, welche nicht mit CD36 defizienten Plättchen (Beschreibung: Kehrel et al 1993, Saelman et al 1994, Kehrel et al 1995) reagierten, aber eine deutliche Anbindung an Kontrollplättchen zeigten. Dieses wurde sowohl mittels Durchflußzytometrie, als auch mit dem Dot-Blot-Verfahren und lmmunpräzipitation bewiesen. Alle Antikörper erkannten gereinigtes CD36. Drei dieser Klone (Klon37, 13 und 7) hemmten die Reaktion von oxidierten Proteinen mit humanen Zellen (s.u.).

### Isolierung von CD36

Die Isolierung von CD36 erfolgte, wie von unserer Arbeitsgruppe beschrieben (Kronenberg et al 1998), über Phasentrennung der Membranproteine mit Triton X-114 und anschließender Affinitätschromatographie an Cholesteryl-Hemisuccinat-Agarose.

### Beispiele für die Herstellung von oxidierten Proteinen

348 µg Protein (handelsübliches Fibrinogen, Humanalbumin, Rinderserumalbumin (BSA) oder Antithrombin) wurden mit 832 µg NaOCl (Natriumhypochlorit) in 1 ml Phosphat-gepufferte Saline mit EDTA-Zusatz (0.1 mM) für 10 Minuten auf Eis inkubiert. Protein und Oxidanz wurden sofort nach Reaktionsende durch eine Gelfiltration bei 4°C über Sepharose G25-Coarse (PD-10 Säule, Amersham Pharmacia) getrennt.

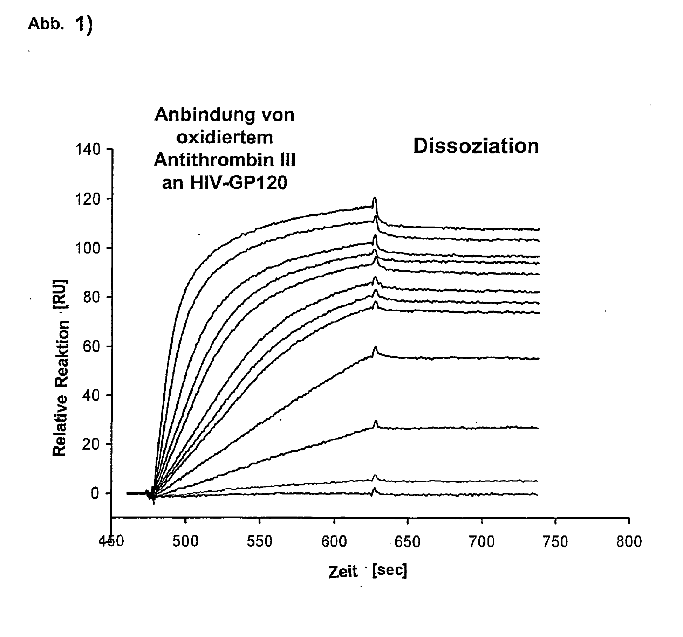

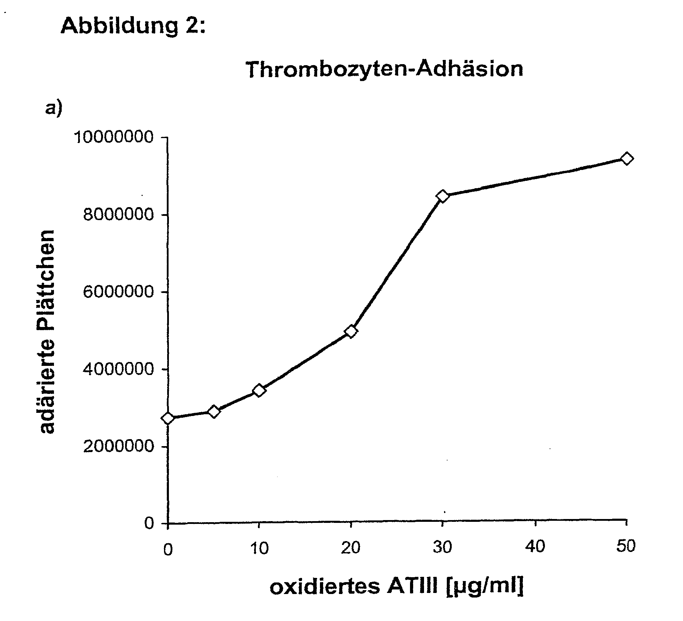

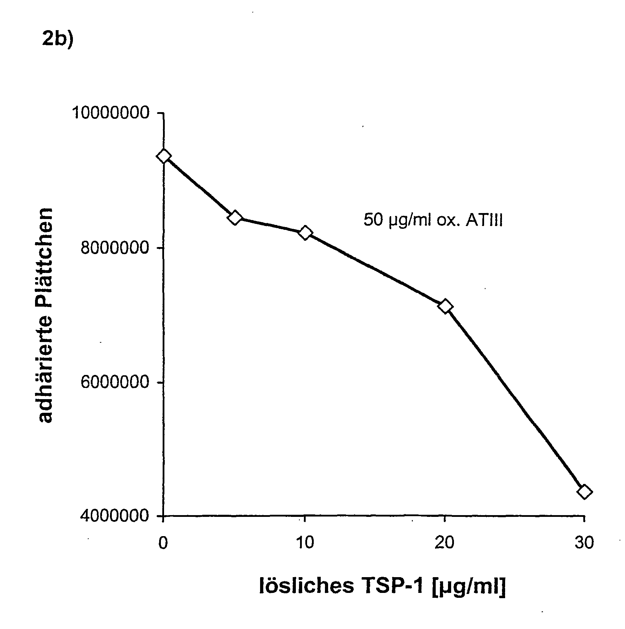

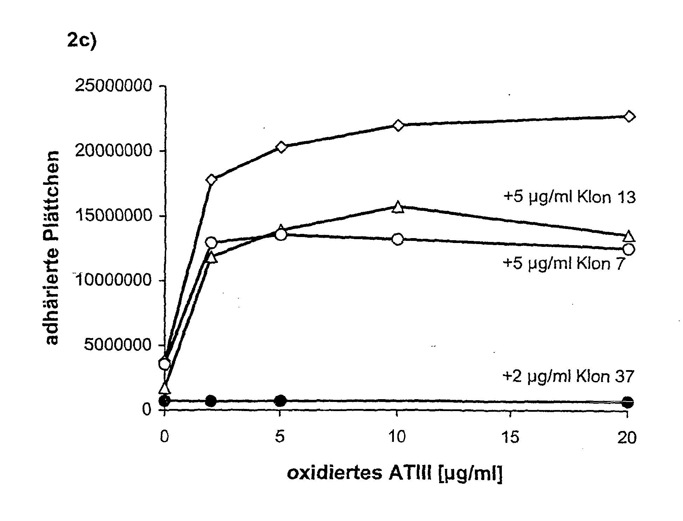

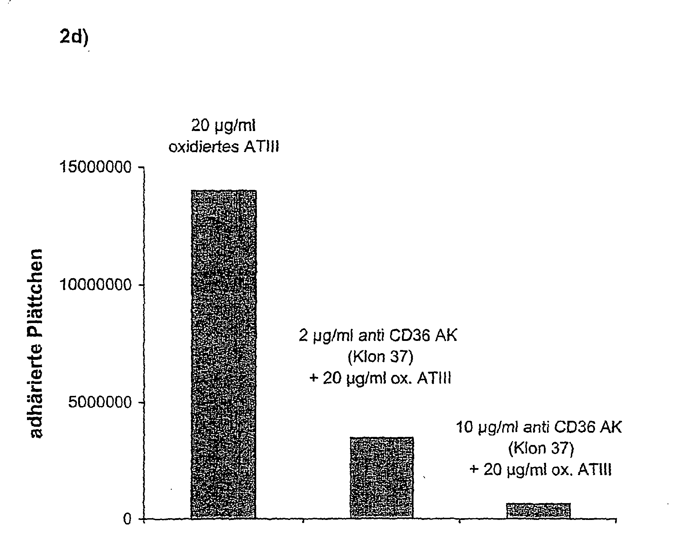

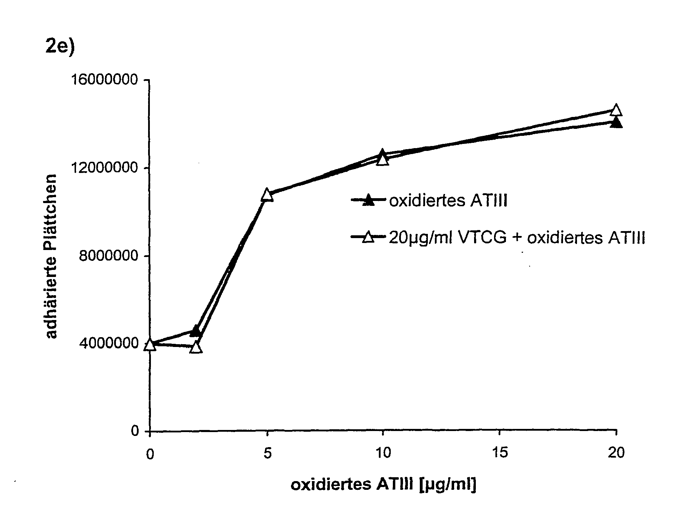

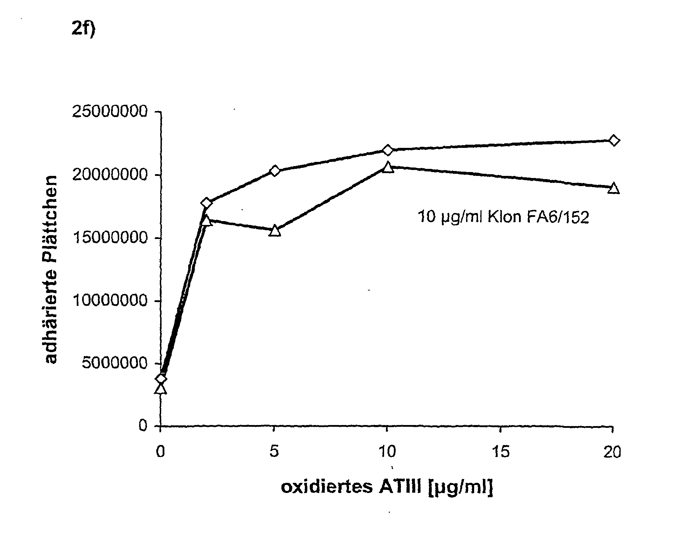

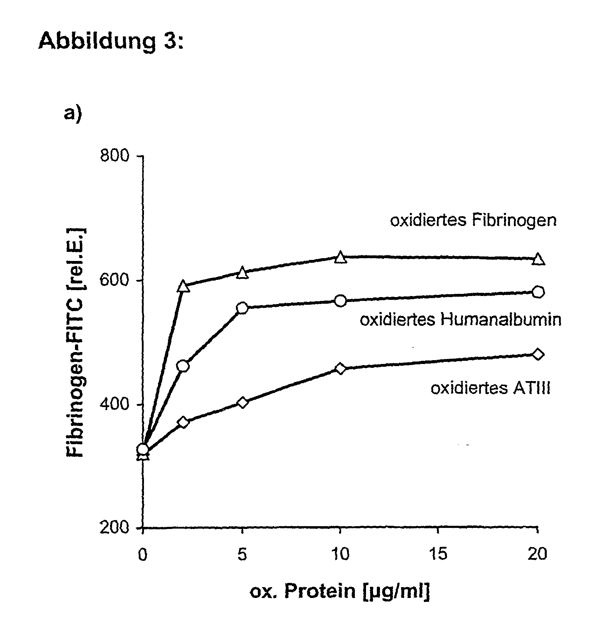

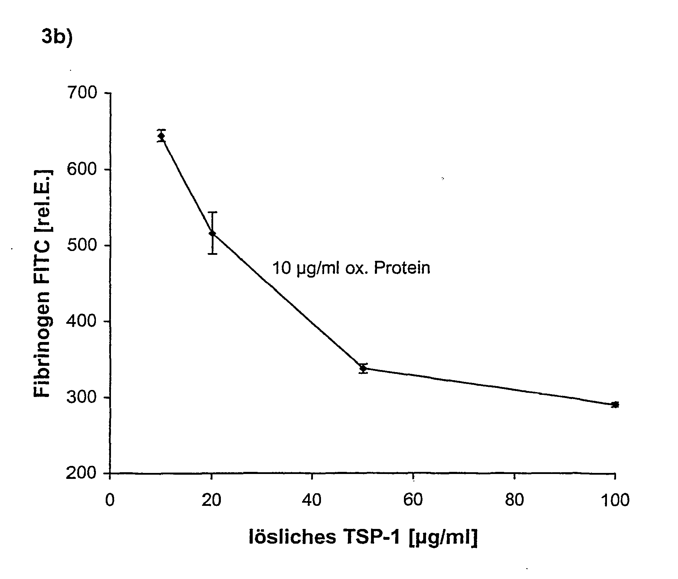

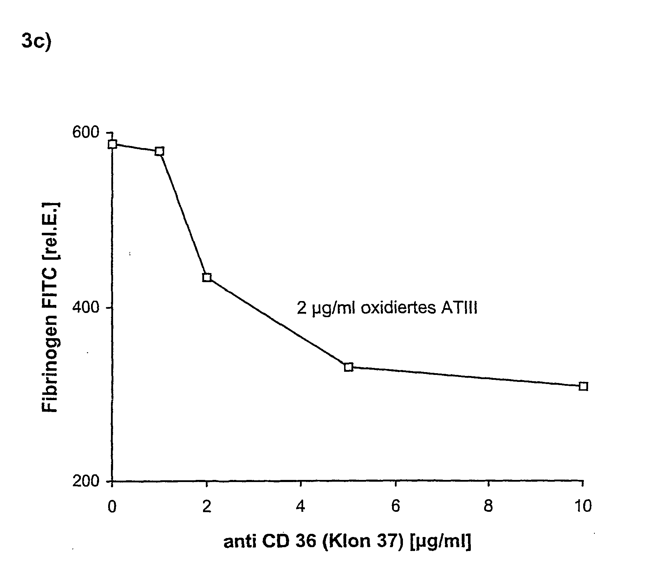

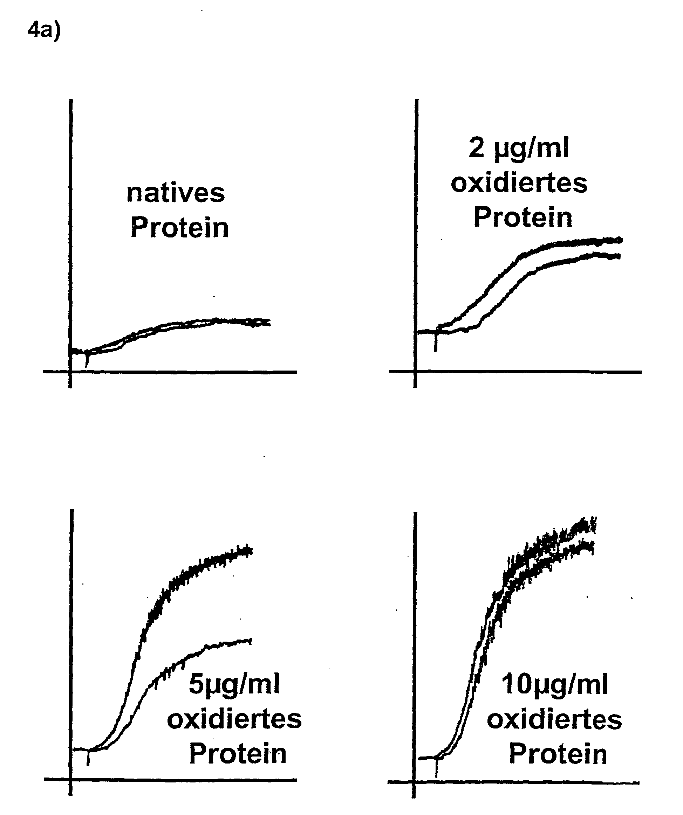

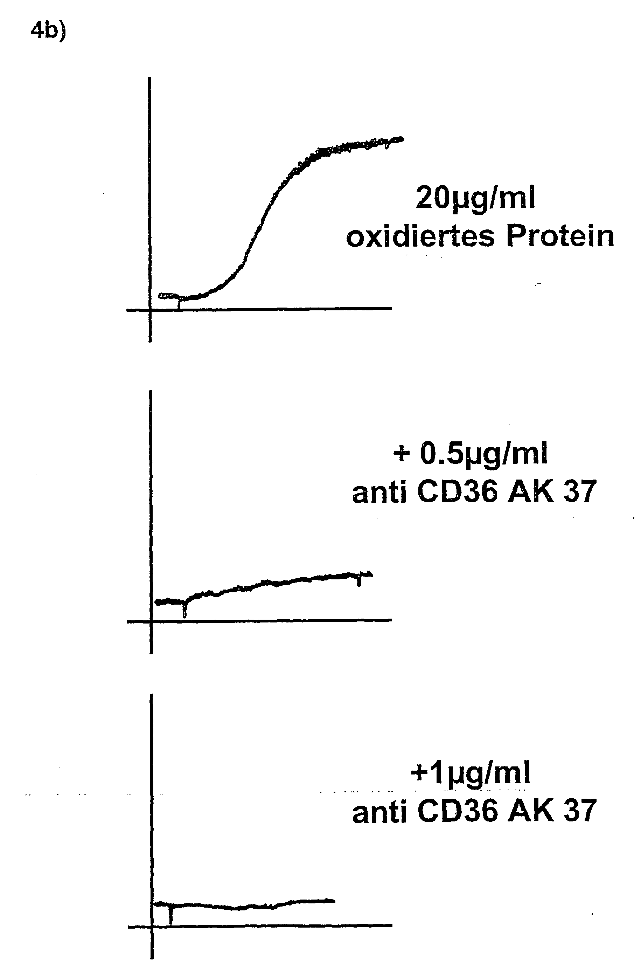

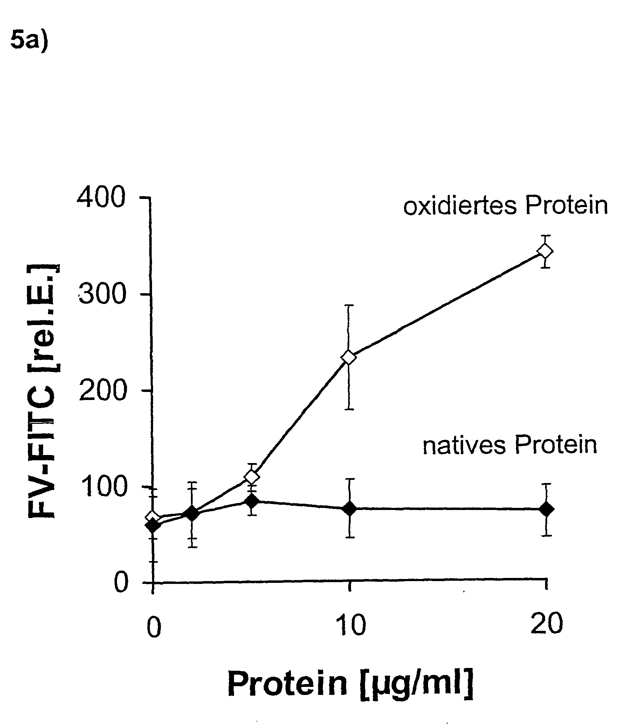

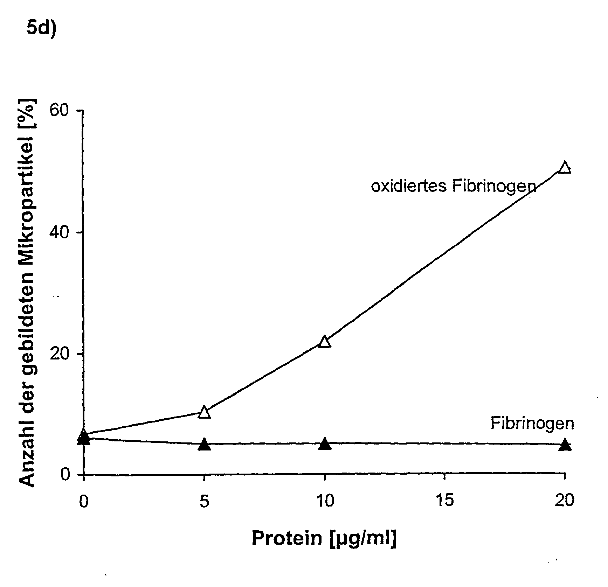

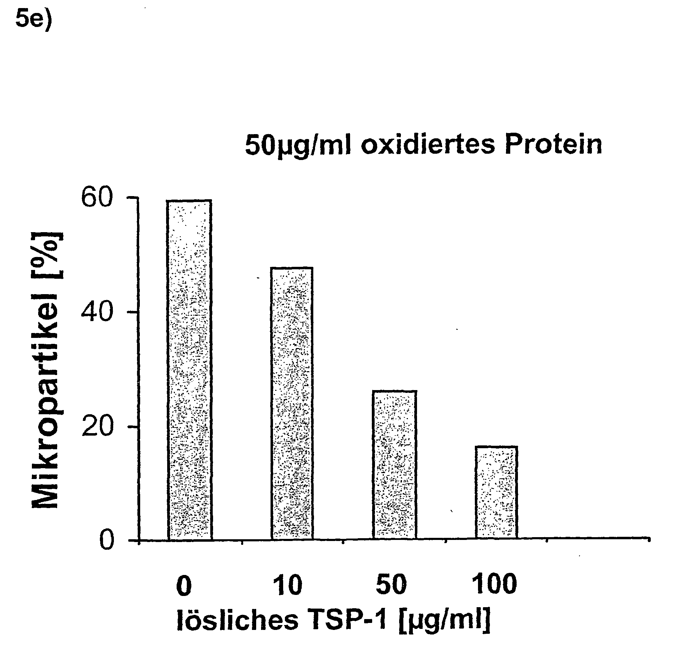

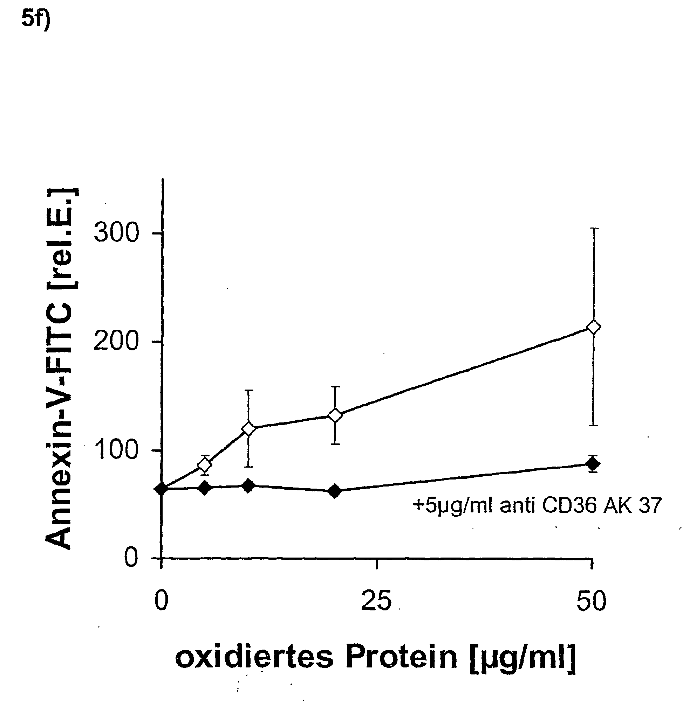

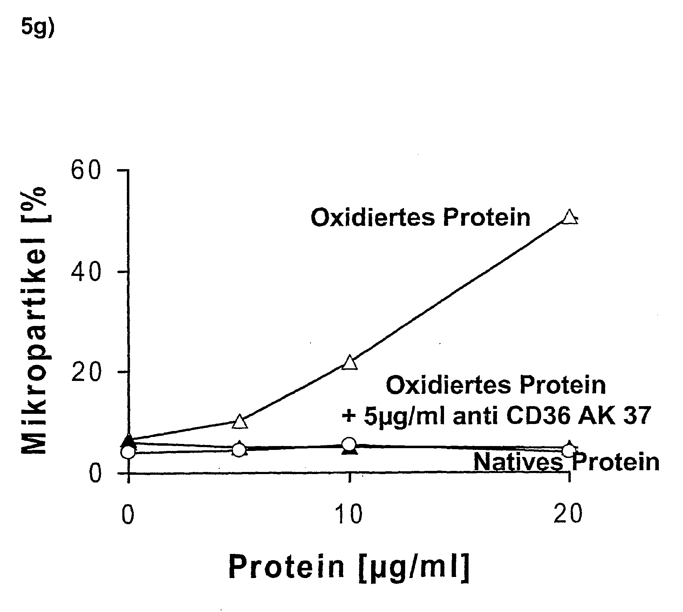

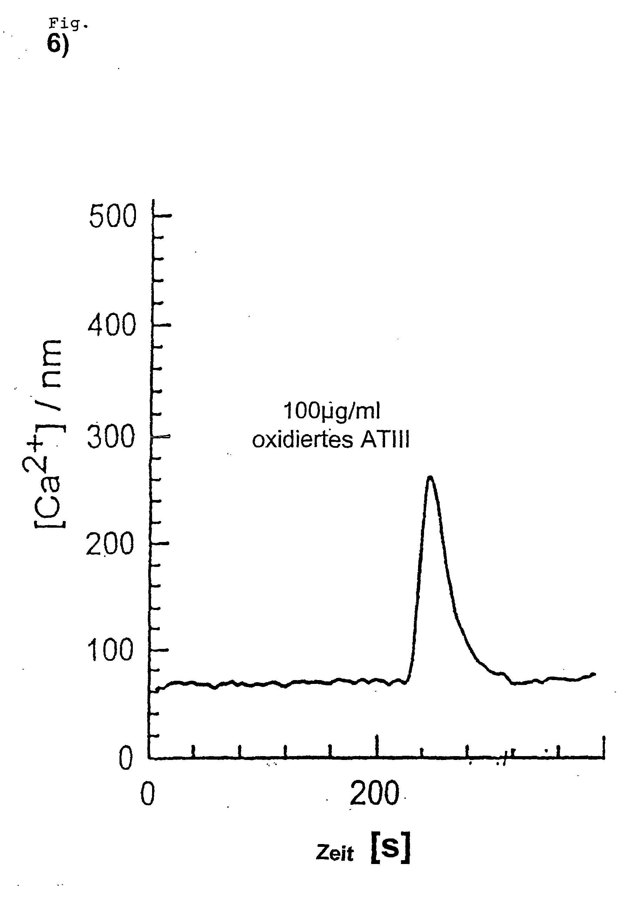

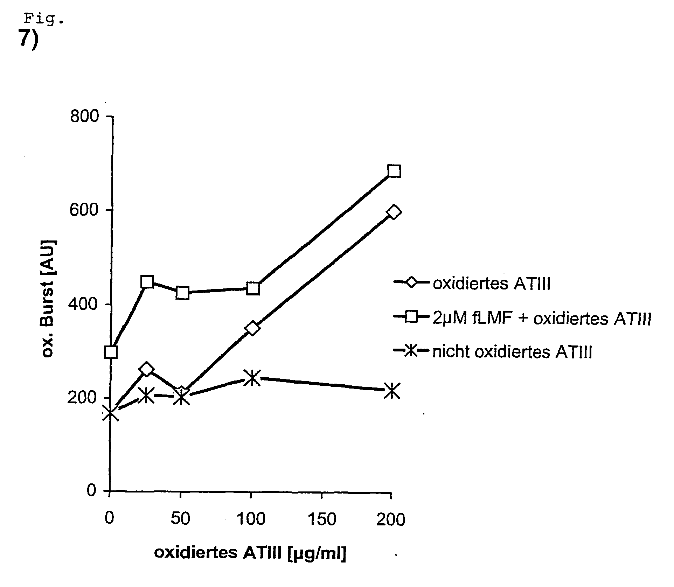

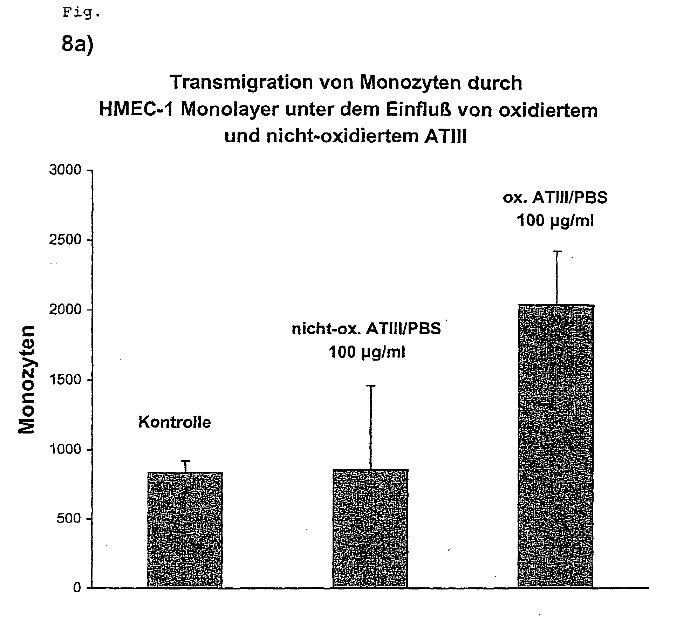

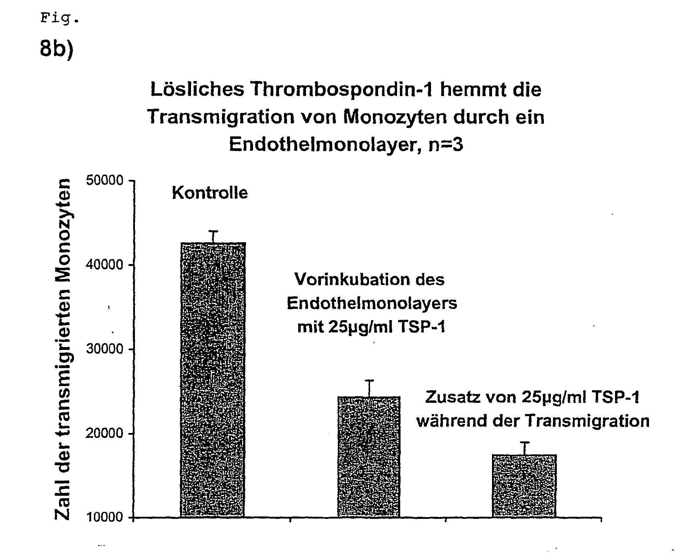

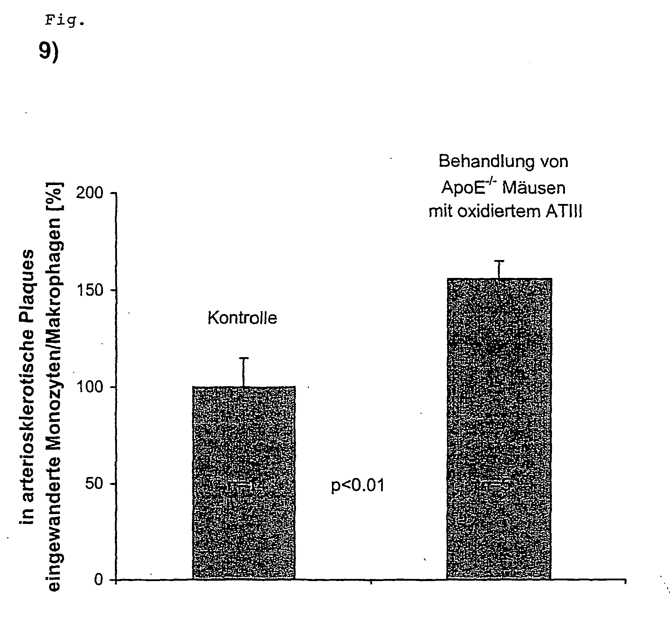

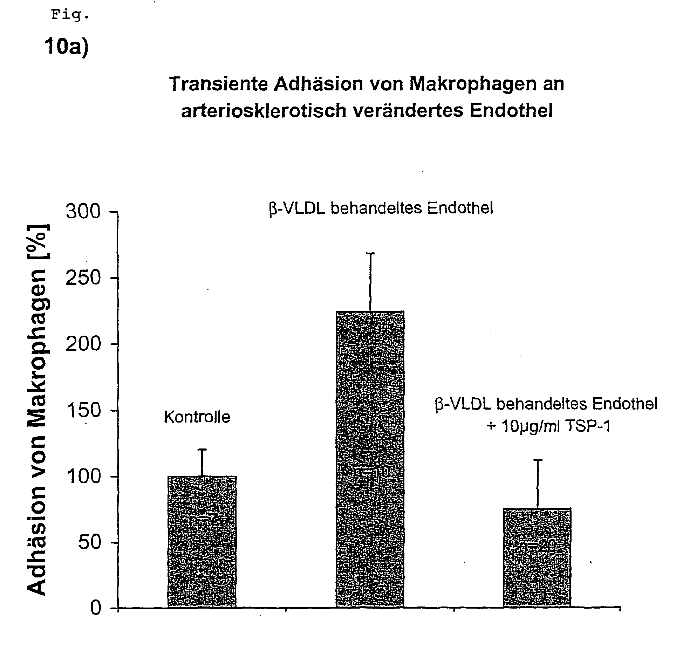

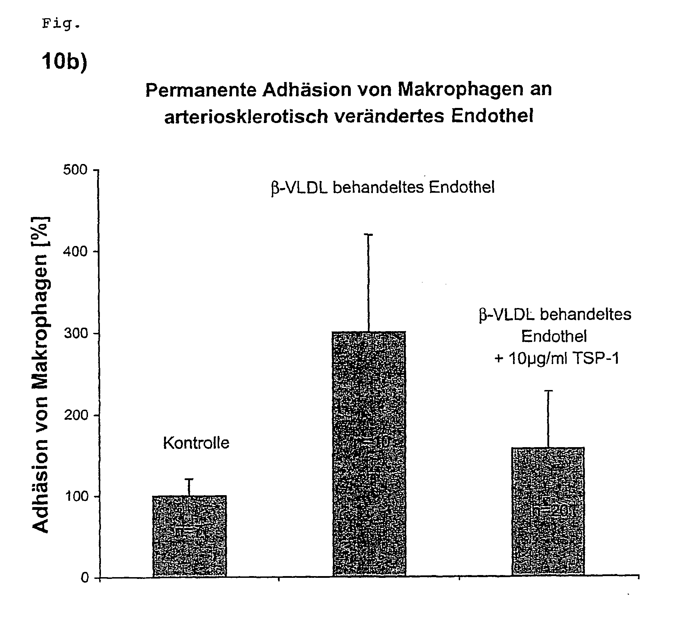

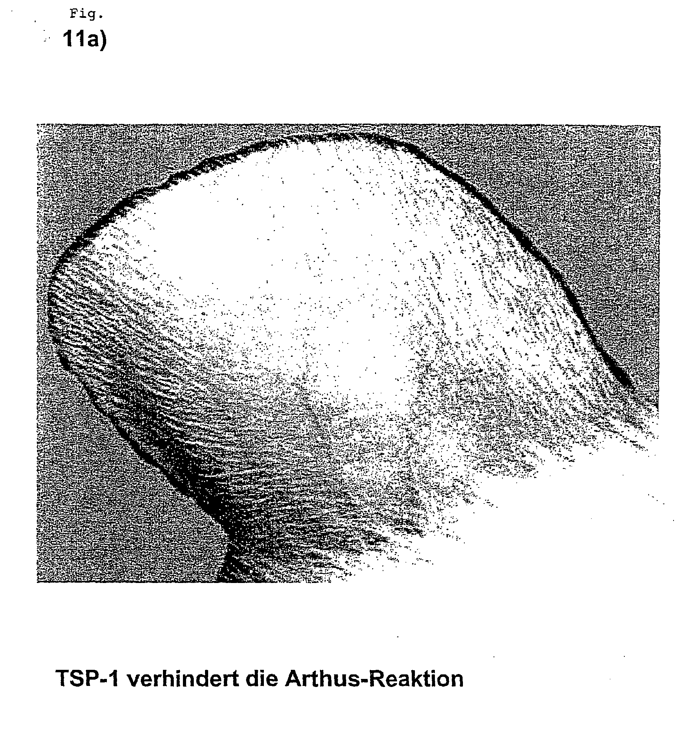

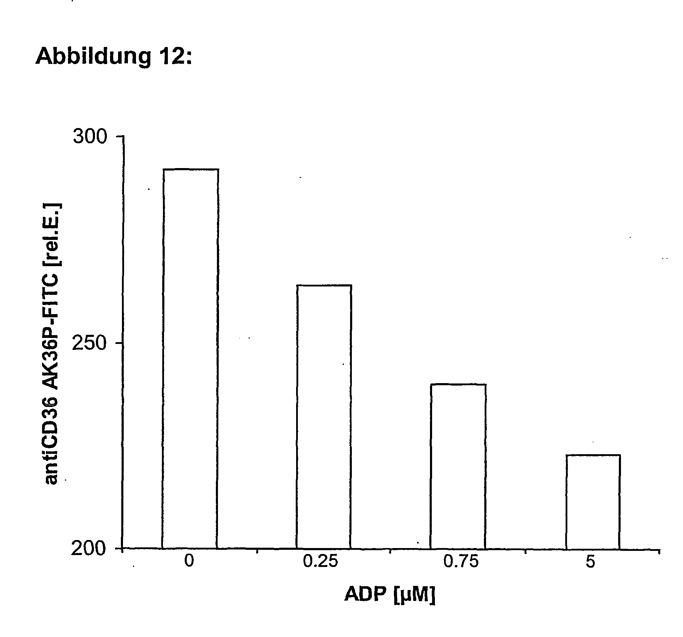

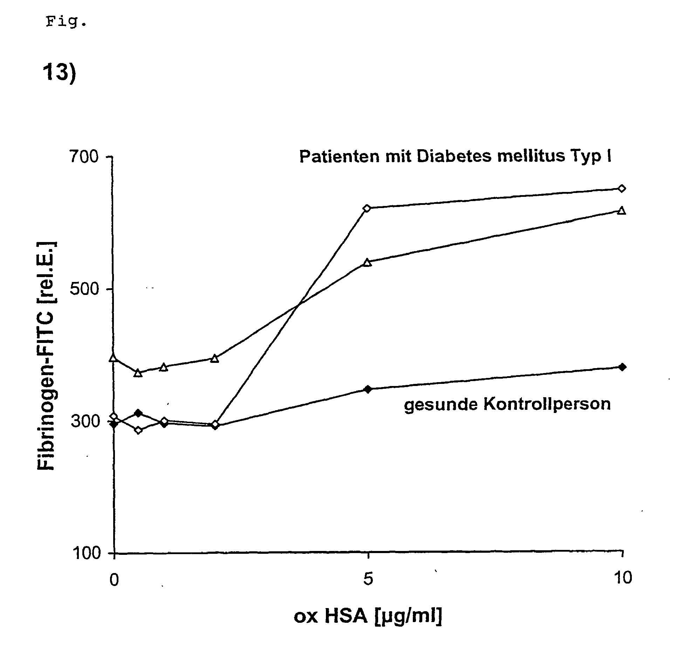

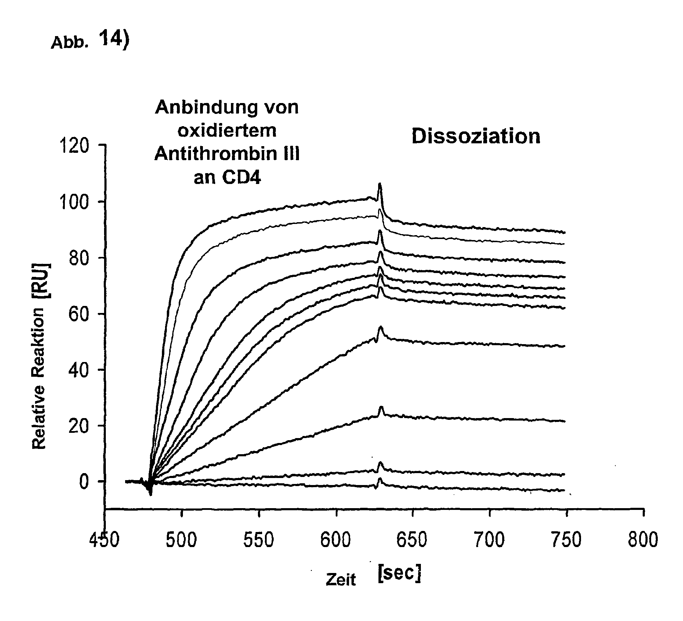

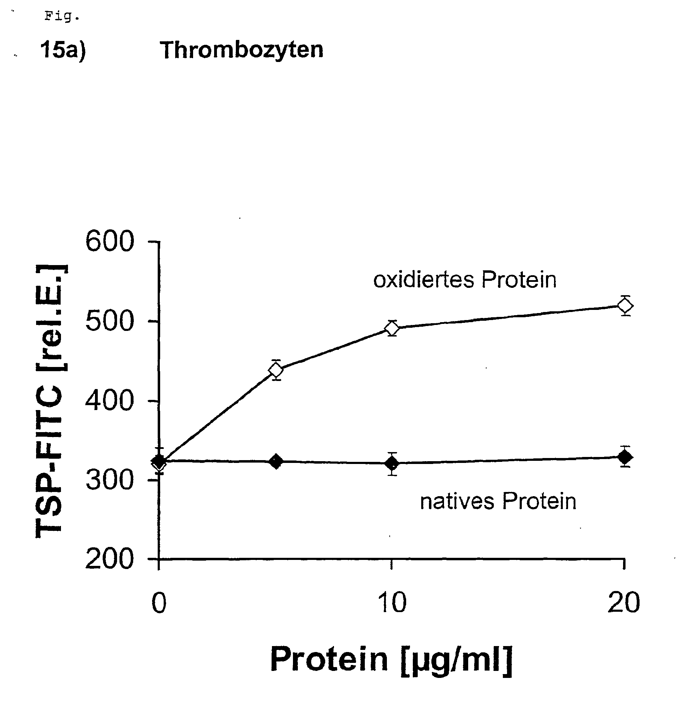

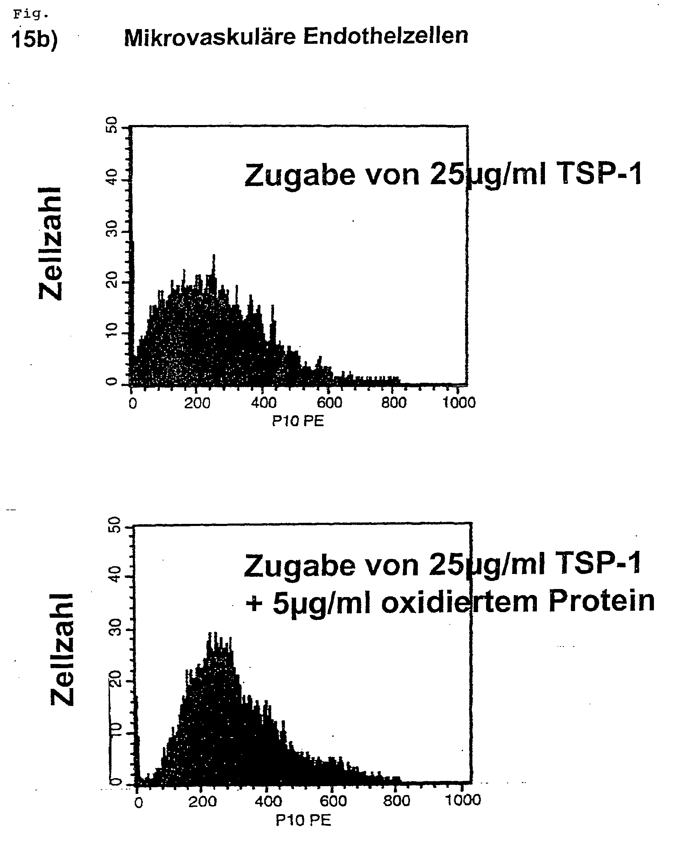

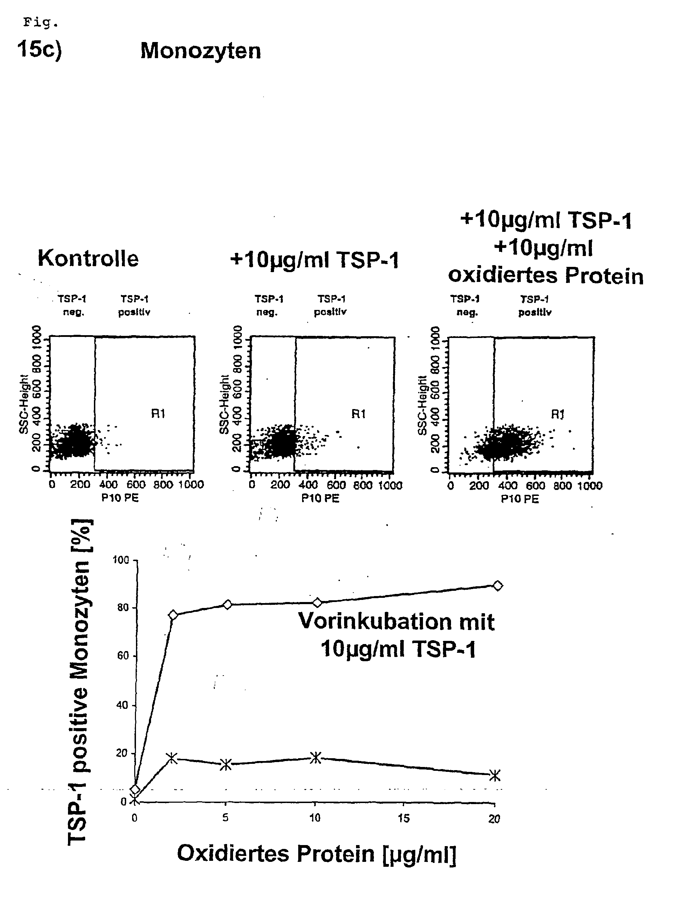

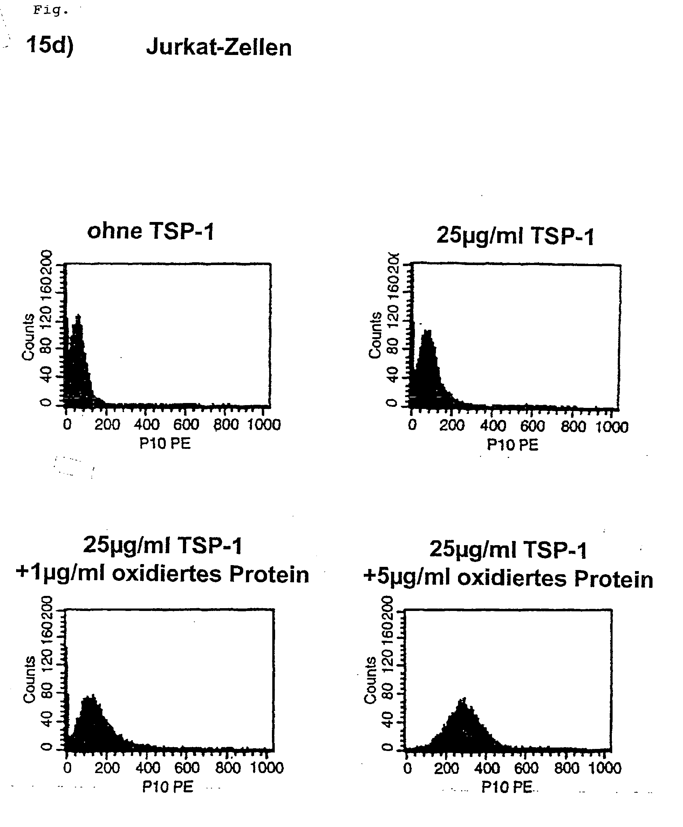

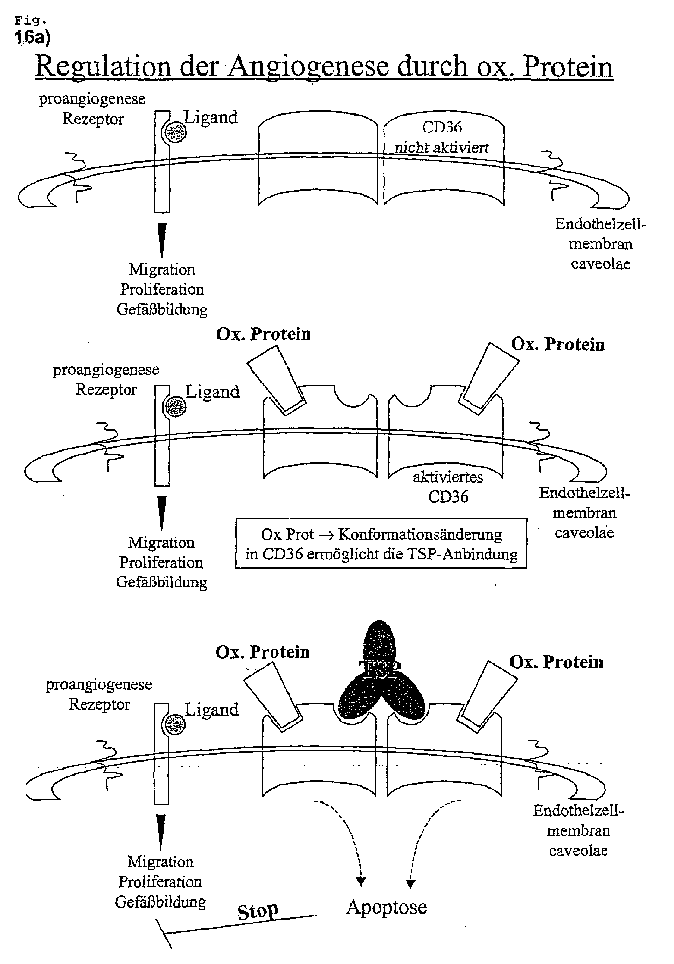

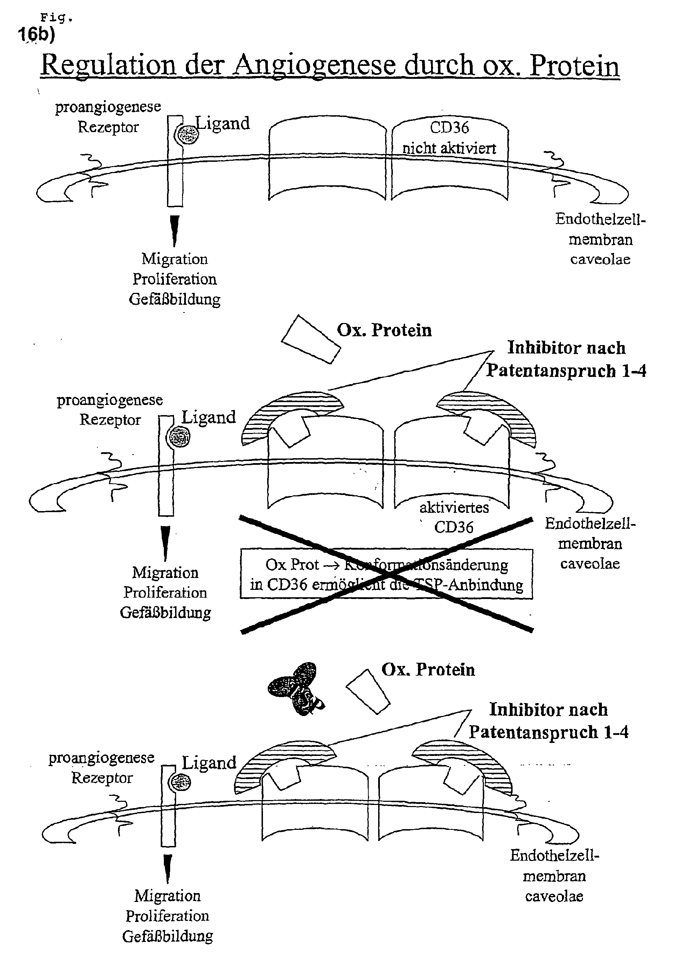

Beispiele für die Wirkung der Erfindung:
1. Oxidierte Proteine (oxFibrinogen, oxAntithrombin III, oxBSA, oxHumanalbumin) binden spezifisch an die CD36-homolge Domäne des HIV GP120 Proteins. (siehe Abbildung 1). Die Interaktion zwischen oxidierten Proteinen und HIV GP120 wurde mittels Plasmon-Resonanz Technik im BIACORE System 2000 bestimmt.
2. Oxidierte Proteine aktivieren Thrombozyten. Diese Aktivierung wird durch Substanzen, die die Interaktion von CD36 mit oxidierten Proteinen hemmen oder mit an CD36 gebundenem Thrombospondin konkurrieren, inhibiert
   a) Oxidierte Proteine (oxFibrinogen, oxAntithrombin III, oxBSA, oxHumanalbumin) verstärken dosisabhängig die Adhäsion von Thrombozyten an Adhäsionsproteine, wie Thrombospondin, Vitronectin, Fibrinogen, Fibrin, Fibronectin und Kollagen (siehe Abbildung 2a).
      Diese Adhäsionsverstärkung wird durch lösliches Thrombospondin-1 inhibiert (siehe Abbildung 2b). Diese Adhäsionsverstärkung wird durch die monoklonalen anti CD36 Antikörper Klon 37, 13 und 7 inhibiert (siehe Abbildung 2c/d, während das VCTG-Peptid, welches die Bindung von Thrombospondin an CD36 hemmt; keinen Einfluß hat (siehe Abb. 2e). Der kommerzielle anti CD36 Antikörper FA6/152 zeigt keine Hemmwirkung (siehe Abb 2f).
   b) Oxidierte Proteine (oxFibrinogen, oxAntithrombin III, oxBSA, oxHumanalbumin) induzieren in Gegenwart von Thrombospondin-1 (10 µg/ml) dosisabhängig die Fibrinogenanbindung an Thrombozyten (siehe Abbildung 3a). Diese Aktivierung wird durch lösliches Thrombospondin-1 in einer Konzentration von > 20 µg/ml inhibiert (siehe Abbildung 3b).
      Diese Aktivierung wird durch die monoklonalen Antikörper gegen CD 36, Klon 37,13 und 7, inhibiert (siehe Abbildung 3c-e)
   c) Oxidierte Proteine (oxFibrinogen, oxAntithrombin III, oxBSA, oxHumanalbumin) lösen in Gegenwart von Thrombospondin-1 (10µg/ml) die Plättchenaggregation aus (siehe Abbildung 4a). Diese Aggregation wird durch die monoklonalen Antikörper gegen CD 36, Klon 37,13 und 7, dosisabhängig inhibiert (siehe Abbildung 4b)
   d) Oxidierte Proteine (oxFibrinogen, oxAntithrombin III, oxBSA, oxHumanalbumin) lösen in Gegenwart von Thrombospondin-1 (10 µg/ml) den prokoagulanten Zustand der Plättchen aus (siehe Abbildungen 5a-c) und führen zur Mikropartikelbildung (siehe Abbildung 5d). Diese Aktivierung wird durch lösliches Thrombospondin-1 in Konzentrationen von ≥ 20 µg/ml inhibiert (siehe Abbildung 5e).
      Diese Aktivierung wird durch die monoklonalen Antikörper gegen CD 36, Klon 37, 13 und 7, dosisabhängig inhibiert (siehe Abbildungen 5f und g).
3. Oxidierte Proteine (z.B. oxFibrinogen, oxAntithrombin III, oxBSA, oxHumanalbumin) aktivieren Monozyten. Diese Aktivierung wird durch Substanzen wie z.B. lösliches Thrombospondin oder monoklonale Antikörper gegen CD36, die die Interaktion zwischen CD36 und oxidierten Proteinen hemmen oder mit an CD36 gebundenem Thrombospondin konkurrieren, dosisabhängig gehemmt.
   a) Oxidierte Proteine (z.B. oxFibrinogen, oxAntithrombin III, oxBSA, oxHumanalbumin) induzieren in Monozyten ein Ca²⁺-Signal (siehe Abbildung 6).
   b) Oxidierte Proteine (z.B. oxFibrinogen, oxAntithrombin III, oxBSA, oxHumanalbumin) induzieren in PMNL den oxidativen Burst (siehe Abbildung 7).
   c) Oxidierte Proteine (z.B. oxFibrinogen, oxAntithrombin III, oxBSA, oxHumanalbumin) induzieren eine erhöhte Transmigration von Monozyten, PMNL und Lymphozyten durch Endothelmonolayer (siehe Abbildung 8a). Diese Reaktion wird durch Substanzen, die die Bindung von CD36 an oxidierte Proteine inhibieren, wie lösliches Thrombospondin oder monoklonale Antikörper gegen CD36, Klone 37, 13 und 7, inhibiert.(siehe Abbildung 8b).
4. Oxidierte Proteine (z.B. oxFibrinogen, oxAntithrombin III, oxBSA, oxHumanalbumin) sind ursächlich an der Entstehung von Arteriosklerose beteiligt.
   a) Oxidierte Proteine (z.B. oxFibrinogen, oxAntithrombin III, oxBSA, oxHumanalbumin) induzieren ein "Homing" von Makrophagen in arteriosklerotische Plaques. Das "Homing" wurde nach Patel et al 1998 durchgeführt. Bei C57BL/6 Mäusen wurde durch intraperitoneale Injektion von Thioglycolat die Wanderung von Monozyten/Makrophagen ins Peritoneum angeregt. Nach 4 Tagen wurde das Peritoneum gewaschen und aktivierte Peritonealmakrophagen gewonnen. In den Proben wurden die Erythrozyten lysiert. Makrophagen wurden in RPMI 1640-Medium aufgenommen und in Zellkulturplatten überführt, um ihnen die Adhäsion zu ermöglichen. Fluoreszenz markierte Mikrosphären (2 µm yellow-green fluorescent latex microspheres, Molecular Probes) wurden zur besseren Aufnahme durch die Phagozyten für 30 Minuten mit 50% Mausserum opsoniert und dann zu den platierten Makrophagen gegeben. Die adhärenten Makrophagen phagozytierten die Mikrosphären. Nicht adhärierte Zellen und Mikrosphären wurden durch Waschen der Platte entfernt. Markierte Makrophagen wurden auf Eis von der Platte abgelöst und in ""Hanks balanced salt solution"(HBSS)resuspendiert. 50 Wochen alten ApoE-defiziente Mäuse wurde je 3 mal je 50 µg oxidiertes Protein, hier oxidiertes Antithrombin III, bzw. Placebo (nur HBSS), intraperitoneal injiziert; 6 Stunden vor intravenöser Injektion von markierten Makrophagen, 2 Stunden nach Makrophageninjektion und 10 Stunden nach Makrophageninjektion. Es wurden 10x10⁶ Makrophagen in 0.2 bis 0.3 ml HBSS suspendiert und in die Schwanzvene gespritzt. 24 Stunden nach Makrophageninjektion wurden die Tiere getötet. Die Herzbasis und die Aorta ascendens wurde in OCT eingebettet, bei -80°C gelagert und 7 µm dicke Kryoschnitte angefertigt. Die fluoreszenzmarkierten Makrophagen von 140 Serienschnitten per Maus, aus einem 1 mm umfassenden Bereich der Aorta ascendens auf der Ebene des Sinus Valsalva wurden gezählt. Im Vergleich zu den Placebo behandelten ApoE^{-/-} Kontrollmäusen stieg die Wanderung von markierten Makrophagen in die arteriosklerotischen Plaques in mit oxidiertem Antithrombin III behandelten Mäusen von 100 ± 15% (n=14) auf 156 ± 9.2% (n = 5) signifikant (p =0.008)("alternate welch test") an. Da diese Einwanderung mit der Entstehung, dem Fortschreiten und der Rupturgefahr des arteriosklerotischen Plaques ursächlich verbunden ist, verdeutlicht dieses Ergebnis die Gefährlichkeit von oxidierten Proteinen in Hinblick auf die Arteriosklerose (Abbildung 9).
   b) Substanzen, die die Interaktion zwischen CD36 und oxidierten Proteinen hemmen oder mit an CD36 gebundenem Thrombospondin konkurrieren, verhindern/reduzieren die pro-arteriosklerotische Wirkung von oxidierten Proteinen. Lösliches Thrombospondin hemmt die Adhäsion von Makrophagen an arteriosklerotisch veränderte Endothelzellen. Dies ist die Voraussetzung für das Einwandern von Makrophagen in arteriosklerotische Plaques. Murine immortalisierte Endothelzellen wurden mit ß-VLDL (50µg Protein/ml)aus Plasma von ApoE-defizienten Mäusen für 6 Stunden stimuliert und dann mit 2x10⁵ peritonealen Monozyten/Makrophagen pro ml bei einer Scherrate von 400s⁻¹ in einer Durchflusskammer superfundiert. ß-VLDL induzierte einen signifikanten relativen Anstieg der rollenden Leukozyten um 224±44% im Vergleich zur Kontrolle. Zusatz von 10 µg/ml lösliches Thrombospondin hemmte diesen Adhäsionszuwachs komplett und inhibierte zusätzlich teilweise die Grundadhäsion der Makrophagen an das Endothel (75±37% Adhäsion im Vergleich zur Kontrolle, n=4-7; p<0.01) (siehe Abbildung 10a). Die durch das ß-VLDL deutlich induzierte permanente Adhäsion der Makrophagen nach einer 5-minütigen Waschperiode wurde durch Thrombospondin-1 ebenfalls reduziert (100±21% Kontrolle vs 300±119% ß-VLDL vs 157±70% ß-VLDL+TSP-1; n=4-7; p<0.01) (Abbildung 10b).
5. Lösliches Thrombospondin-1 inhibiert Entzündungsreaktionen in vivo. Im Ohr von Balb/c-Mäusen wurde durch lokale Injektion von anti-BSA zum Zeitpunkt 0 und gleichzeitiger Injektion von FITC gekoppeltem BSA ins Peritoneum eine Arthus-Reaktion ausgelöst. Kontrolltieren (Negativkontrollen) wurde nur FITC (ohne BSA) ins Peritoneum injiziert. Nach ca. 6 Stunden zeigte sich eine deutlich ausgeprägte Entzündungsreaktion mit Anschwellung des Ohrs (Oedem), FITC-Einlagerung, Einwanderung von PMNL und petechialen Einblutungen ins Gewebe bei den mit anti-BSA und BSA-FITC behandelten Tieren. 18 Mäuse wurden zusätzlich zum Zeitpunkt 0 und nach 0+3 Stunden je 50µg Thrombospondin-1 in Puffer (PBS) intraperitoneal injiziert. 16 Kontrollmäuse erhielten zum Zeitpunkt 0+3 Stunden nur PBS. Die Arthus-Reaktion wurde durch Thrombospondin fast vollständig verhindert. Mit Thrombospondin behandelte Mäuse zeigten signifikant geringere FITC-Einlagerungen, signifikant geringere Ohrdicken (weniger Oedeme) und fast keine Petechien im Vergleich zu PBS behandelten Kontrolltieren (siehe Abbildungen 11a-c).
6. Beispiele für die Quantifizierung von oxidierten Proteinen Herstellung von monoklonalen Antikörpern, die durch Oxidierungsprozesse direkt oder indirekt veränderte Epitope auf Proteinen oder Peptiden erkennen: Humanalbumin, Antithrombin III und Fibrinogen wurden, wie in dieser Erfindungsmeldung beschrieben, mit HOCI oxidiert und 8-12 Wochen alte Balb/c Mausweibchen damit immunisiert. Die Hybridomherstellung und -kultur erfolgte nach klassischem Verfahren. Überstände von Hybridomen wurden auf die Produktion von lgG bzw. lgM getestet. lgG-positive Zellkulturüberstände wurden auf positive Reaktion mit oxidiertem Protein, bei gleichzeitig negativer Reaktion mit dem nicht veränderten Ausgangsprotein getestet. Klone, die Antikörper gegen oxidiertes Protein (oxHumanalbumin, oxAntithrombin III, oxFibrinogen) produzierten und gleichzeitig nicht mit nicht oxidiertem Mutterprotein reagierten, wurden auf Kreuzreaktion mit oxidierten anderen Proteinen und Peptiden getestet.
   Quantifizierung oxidierter Proteine mit Hilfe von monoklonalen Antikörpern, wie oben beschrieben:
   Eine solche Quantifizierung ist leicht mit Verfahren wie ELISA, RIA, quantitativer Durchflußzytometrie auf Zelloberflächen und ähnlichen Routineverfahren möglich.
   z.B.: Quantifizierung von oxidiertem Humanalbumin mittels ELISA. Ein polyklonaler Antikörper aus dem Kaninchen gegen Humanalbumin (Herstellung - Routineverfahren) wurde als Fängerantikörper an den Boden einer ELISA-Platte (Nunc-Maxisorb) gebunden. Die Platte wurde mit PBS pH 7,4, 0,5% Tween 20 gründlich gewaschen und Plätze auf der Plastikoberfläche mit 3% BSA für 1 Stunde bei Raumtemperatur (RT) geblockt. Die Platte wurde wieder gewaschen und anschließend mit unterschiedlich verdünnten Plasmen, Seren, Überstanden aus Blutprodukten (z.B. Thrombozytenkonzentraten, Erythrozytenkonzentraten, FFP) oder Pufferlösungen, denen oxHumanalbumin in definierter Menge zugesetzt wurde, für 1 Stunde bei RT inkubiert. Probenmaterial bzw. Standardlösungen wurden entfernt, die Platte gründlich gewaschen und mit dem oben beschriebenen monoklonalen Antikörper, der oxidiertes Humanalbumin erkennt und der mit Biotin markiert worden war, in einer Verdünnung von 1:15000 in PBS, 1 % NGS (normal goat serum) inkubiert. Die Platte wurde wiederum mehrfach gründlich gewaschen und mit Streptavidin-Peroxidase inkubiert (1 Stunde, RT). Nach erneutem Waschen der Platte wurde diese mit Substratlösung (100µl/well) (20mg ortho-Phenyldiamin, 5% H₂O₂ in einem Puffer aus 12,15ml 0,1 M Zitronensäure und 12,85ml 0,2 M Na₂HPO₄ plus 25ml-H₂O dest.) versetzt. Die Extinktion bei 405nm gemessen im ELISA-Photometer spiegelt die Menge an oxidiertem Humanalbumin wieder. Die Reaktion wurde mit 50µ/well 4 n H₂SO₄ gestoppt und die Extinktion bei .490nm gemessen.
   HOCl oxidiertes Humanalbumin in der Messprobe wurde über die Eichreihe quantifiziert. Für HOCI-modifiziertes Fibrinogen und HOCI-modifiziertes Antithrombin III wurde analog verfahren.
7. Beispiel für die Erfassung des Aktivierungszustands des Rezeptors für oxidierte Proteine, CD36
   Herstellung von polyklonalen Antikörpern gegen Threonin(92) phosphoryliertes CD36.
   Die Peptide (15 AS) (1) Lys, Gln, Arg, Gly, Pro, Tyr, Thr, Tyr, Arg, Val, Arg, Phe, Leu, Ala, Lys und (2) Lys, Gln, Arg, Gly, Pro, Tyr, PhosphoThr, Tyr, Arg, Val, Arg, Phe, Leu, Ala, Lys (wie Peptid (1), aber am Threonin phosphoryliert) wurden synthetisiert und an KLH (Keyhole Limpet Hemocyanin) gekoppelt. Polyklonale Antikörper aus dem Kaninchen wurden mit diesen gekoppelten Peptiden nach Standardverfahren hergestellt. Dazu wurden Kaninchen (New Zealand, white) mit 250 µg Peptid 1-KLH, bzw. Peptid 2-KLH plus Zusatz von komplettem Freund'schem Adjuvanz s.c. grundimmunisiert und mit 250 µg Peptid 1-KLH, bzw. Peptid 2-KLH mit Zusatz von Alu-Gel-S (1.3% Aluminiumhydroxid in Wasser, SIGMA) 3 mal "geboostert". Den Tieren wurde aus der Ohrvene Blut entnommen, Serum hergestellt und dieses auf Antikörper gegen die zur Immunisierung verwandten Peptide getestet. Der Antikörper gegen das phosphorylierte CD36-Peptid wurde an einer Affinitätssäule mit nicht-phosphoryliertem CD36-Peptid kreuzabsorbiert, so dass das erhaltene Antikörpergemisch nur noch Antikörper enthielt, die spezifisch phosphoryliertes, nichtaktiviertes CD36 erkennen (AK CD36P). (Die Herstellung von spezifischen **monoklonalen Antikörpern gegen** Threonin-phosphoryliertes/-dephosphoryliertes CD36 ist mit diesen Peptiden analog zur beschriebenen Herstellung von anti-CD36-Protein AK möglich.)
   Beide polyklonalen Antiseren reagierten mit CD36 auf der Plättchenoberfläche in der Durchflußzytometrie. Während Antikörper "CD36P" gegen phosphoryliertes CD36 das CD36 bevorzugt auf nicht aktivierten Thrombozyten erkennt, erkennt Antikörper "CD36-gesamt" nicht-phosphoryliertes CD36 und phosphoryliertes CD36. Bei Aktivierung der Thrombozyten wird CD36 dephosphoryliert und die Bindungsfähigkeit für Antikörper "CD36P"" nimmt ab (siehe Abbildung 12). Durch quantitative Durchflußzytometrie mit beiden Antikörpern ist eine Berechnung des Anteils an aktiviertem CD36 möglich.
8. Der Organismus von Patienten mit Typ I Diabetes ist besonders empfindlich für oxidierte Proteine:
   z.B.: Thrombozyten von Patienten mit Diabetes Typ I reagieren sensitiver auf oxidiertes Protein als Agonist als Thrombozyten von gesunden Kontrollprobanden. Die aktivierungsabhängige Fibrinogenbindung lässt sich auf Thrombozyten von Diabetikern mit geringeren Konzentrationen an ox. Protein auslösen, als auf Thrombozyten von Kontrollprobanden (siehe Abbildung 13).
9. Oxidierte Proteine verhindern eine HIV-Infektion. Oxidierte Proteine (getestet wurden oxAntithrombin III und oxHumanalbumin) binden mit hoher Affinität sowohl an HIV-GP120, als auch an seinen Rezeptor CD4.
   z.B.: Die Bindung von oxidiertem Antithrombin III und oxidiertem Humanalbumin an HIV-GP120 und an CD4 wurde im BIACORE 2000 System nachgewiesen. Laufpuffer: 25 mM Tris; 100 mM NaCl pH7,4; 1 mM CaCl₂; 1 mM MgCl₂; 0,005% Surfactant P-20.
   Protein HIV-GP120: c = 100µg/ml, 200µl
   Lagerungspuffer: Tris/HCl, NaCl pH 7,6
   Protein CD4: c=63µg/ml, 318µl
   Lagerungspuffer: 10mM Tris; 300mM NaCl pH 8
   Protein ox-Antithrombin III: c=1mg/ml, 120µl
   Protein ox-Humanalbumin: c=1mg/ml, 120µl
   C1-Chip (BIACORRE AB)
   Aminkopplungskit (BIACORE AB)
   HIV-GP120 und CD4 wurden auf der C1-Sensoroberfläche immobilisiert. Dabei wurden 10mM NaAc pH 4 als Kopplungspuffer verwendet.
   Kopplungsbedingungen: HIV-GP120: 20µl; 10µg/ml GP120 in 10mM
   NaAc pH 4; immobilisierte Menge: 1158 RU, 300pg
   CD4: 30µl; 6,3µg/ml CD4 in 10mM NaAc pH 4; immobilisierte
   Menge: 568 RU, 148p
   Die Chip-Oberfläche wurde mit BSA abgesättigt und die Anbindung der oxidierten Proteine untersucht. Dazu wurden z.B. 50µl oxidiertes Antithrombin III in unterschiedlichen Konzentrationen bei einer Flussrate von 20µl/min injiziert. Die Proteinlösungen wurden mit dem Ladepuffer verdünnt. Mit zunehmender Konzentration an oxidiertem Antithrombin III nehmen die resultierenden Signale zu. Abbildung 14 Overlay-Plot von 12 Sensorgrammen, welche die Anbindung von oxidiertem Antithrombin III an immobilisiertes CD4 und die Dissoziation zeigen
   Die quantitative Auswertung ergab für die Bindung von
   a) oxidiertem Antithrombin III an HIV-GP120:
      Kₒₙ (1 /Ms): 6,38 x 10⁵
      K_{off} (1 /s): 4,44 x 10⁻⁴
      und eine K_{D}[M] von 7,01 x 10⁻¹⁰,
   b) oxidiertem Antithrombin III an CD4:
      Kₒₙ (1/Ms): 7,13 x 10⁵
      K_{off} (1/s): 1,12 x 10⁻³
      und eine K_{D}[M] von 1,63 x 10⁻⁹,
   c) nicht modifiziertes Antithrombin III band weder an HIV-GP120, noch an CD4. Oxidiertes Humanalbumin band mit noch höher Affinität an HIV-GP120 und CD4 als ox. Antithrombin III. Nicht oxidiertes Humanalbumin band weder an HIV-GP120, noch an CD4. Oxidiertes Protein inhibiert die HIV-1 Infektion von monozytären Zellen aus periphärem Blut (PBMC).
   PHA-aktivierte PBMC wurden zusammen mit negativem humanem Serum 1:100 (Negativ-Kontrolle), mit neutralisierendem V3-loop spezifischen Antikörpern (Positivkontrolle), mit oxidiertem Protein (150µg/ml) und einem CCRS-tropen HIV-1 Primärisolat (903) aus einem Patienten inkubiert und nach 5 Tagen die Virus-Produktion mittels P24-ELISA untersucht. Dazu wurden frisch PHA-aktivierte PBMC in RPMI 1640 Medium plus 20% FKS plus 100 U/ml IL-2 in einer Zellkonzentration von 2x10⁶ Zellen/ml aufgenommen und mit jeweils 200.000 Zellen/well/100µl auf einer 96 well-Flachbodenplatte verteilt. Auf Inhibition zu testende Substanzen,
   Postivkontrolle: neutralisierender humaner anti V3-loop Antikörper (1:100)
   Negativkontrolle: negatives Humanserum 1:100
   Verum: oxidiertes Protein (150µg/ml)
   wurden in RPMI-Medium den Zellen zugesetzt und für 30 Minuten bei 37°C/5% CO₂ inkubiert. Anschließend wurde das HIV-1 Virus zu den Ansätzen gegeben: jeweils 10µl/well aus HIV-1 Primärisolat 903-Überstand (CCR5-trop) mit 20.000 TCID₅₀ (50% tissue culture infective dose)/ml ≅ 1000 TCID₅₀/ml im well. Diese Ansätze wurden über Nacht bei 37°C/5% CO₂ inkubiert. Am nächsten Tag wurden die Zellen 3 dreimal mit RPMI 1640 gewaschen und neues Kulturmedium zugegeben. Am fünften Tag nach der Infektion wurden die P24-ELISA Untersuchungen durchgeführt.
   P24-ELISA:
   Die verwendeten anti P24 Antikörper (11-G7 [Niedrig, Berlin] und D7320 [Biochrom]) erkennen das P24-Protein der verwendeten Primärisolatvariante 903. Maxi-Sorb-ELISA-Platten (Nunc) wurden mit diesen Antikörpern über Nacht beschichtet. Der Virus-Überstand aus dem Inhibitionsversuch wurde mit 1 % Triton X-100 inaktiviert. Der inaktivierte Virus-Überstand und der alkalische-Phosphatase-konjugierte-Detektions-Antikörper(BC 1071-AP[Aalto]) wurden, nach Waschen der beschichteten Wells mit PBS, gemeinsam in die Wells überführt und dort für 5 Stunden bei 37°C inkubiert. Die Wells wurden wieder mit PBS gewaschen, gelöstes Substrat für die alkalische Phosphatase p-Nitrophenyl-Phosphat (Sigma) in die Wells gegeben und die Farbentwicklung nach 20 Minuten bei 405 nm im ELISA-Photometer gemessen. Die Parallelwerte in dem verwendeten P24-ELISA schwankten bis zu 0.02 optische Dichte (oD) Einheiten um einen gemeinsamen Mittelwert. Während die oD 405 nm für die Negativkontrolle ≅ keine Inhibition bei 0.8 lag, reduzierte der neutralisierende Antikörper (Positivkontrolle) die oD auf 0.12. 150µg/ml oxidiertes Protein reduzierte die oD auf 0.10. Durch Zugabe von oxidierten Proteinen konnte die HIV-1 Infektion der PBMCs effektiv gehemmt werden.
10. Oxidierte Proteine induzieren die TSP-Bindung an Zellen
   Oxidierte Proteine (hier als Beispiel verwendet oxHumanalbumin, oxAntithrombin III und oxFibrinogen) induzieren spezifisch und dosisabhängig die Bindung von TSP-1 an CD36 haltige Zellen (siehe Abbildungen 15a-d)

Gegenstande der vorliegenden Erfindung ergeben sich aus den Patentsprüchen.

In einer bevorzugten Ausführungsform der Erfindung enthalten die Arzneimittel Antikörper, die die Bindung von oxidierten Proteinen an CD36 hemmen, wobei widerum besonders bevorzugt monoklonale Antikörper einschließlich Antikörperfragmenten wie der F(ab)₂, F(ab), oder der Antikörper-Erkennungsregion, enthalten sind.

In weiteren bevorzugten Ausführungsform enthält das Arzneimittel Proteine ,die die Bindung von CD36 an oxidierte Proteine hemmen oder mit an CD36 gebundenem Thrombospondin, konkurrieren, wobei es sich um lösliches Thrombospondin, handelt.

Zum anderen ist Gegenstand der vorliegenden Erfindung das erfindungsgemäße Arzneimittel zur Verwendung in der Prophylaxe von Thrombosen, insbesondere bei entzündlichen Erkrankungen, in der Unterstützung einer antithrombotischen, Therapie, in der verhinderung einer Transplantatabstoßung, in der Verhinderung einer transplantationsbedingten Arteriosklerose, , in der Verhinderung der Entwicklung und des Fortschreitens von arteriosklerotischen (atherosklerotischen) Erkrankungen, in der Behandlung von chronischen Entzündungsreaktionen, in der Verhinderung einer frühen Gefäß-Reocclusion nach Bypassoperation, Stent, PTCA, o.ä., in der Verhinderung einer Gefäß-Restenose nach Bypassoperation, Stent, PTCA, o.ä., in der Verhinderung von Reperfusionsschäden, wie, aber nicht beschränkt auf, Myokardischemie, Organtransplantation, Schlaganfall, peripherer Verschlußkrankheit, nach operativen Eingriffen oder/und Multiorganversagen nach erfolgreicher Wiederbelebung, in der Verhinderung von Gefäßschädigungen, insbesondere bei Patienten mit Diabetes mellitus, in der Verhinderung einer durch oxidierte Proteine über CD36/TSP-1 ausgelösten Hemmung der Endothelproliferation und Angiogenese, sowie in der Unterstützung der Wundheilung.

Wiederum ein weiterer Gegenstand der vorliegenden Erfindung ist ein Verfahren zur Quantifizierung von oxidierten Proteinen (insbesondere oxAntithrombin III, oxHumanalbumin oder oxFibrinogen), jeweils einzeln oder gesamt zur Qualitätskontrolle von Blutprodukten.

Erfinderungsgemäße Arzneimittel sind auch dadurch gekennzeichnet, dass sie weitere pharmazeutisch akzeptable Hilfs- oder/und Trägersubstanzen enthalten können. Die erfindungsgemäßen Arzneimittel sind bevorzugt zur lokalen, intradermalen, oberflächlichen, intraperitonealen, intravenösen, oralen oder intramuskulären Verabreichung geeignet oder sie können über Vesikel verabreicht werden. Desweiteren ist es bevorzugt, dass die erfindungsgemäßen Arzneimittel weitere Substanzen, wie z.B. Antibiotika, Immunsuppressiva oder Interaktionspartner von oxidierten Proteinen im Körper enthalten. Durch den Zusatz solcher Substanzen können die Wirkungen des erfindungsgemäßen Arzneimittels weiter gefördert und unterstützt werden.

Die im Rahmen der vorliegenden Erfindung oxidierten Proteine oder Peptide werden gemäß der vorliegenden Erfindung bevorzugt durch Reaktion mit HOCI oder Peroxynitriten erzeugt.

### Legenden zu den Abbildungen:

Abbildung 1: Oxidierte Proteine binden an CD36-homolge Domäne im HIV-1 GP120 Protein
   1) Overlay-Plot von 12 Sensorgrammen, welche die Anbindung von oxidiertem Antithrombin III an immobilisiertes HIV-GP120 und die Dissoziation von oxidiertem Antithrombin III zeigen. HIV-GP120 ist immobilisiert (300pg); die Konzentration an oxidiertem Antithrombin III wurde variiert (von unten nach oben: OnM; 1 nM; 5.1 nM; 10.2nM; 17nM; 20.4nM; 23nM; 34nM; 40.8nM; 51 nM; 85nM; 119nM). Mit steigender Konzentration an oxidiertem AT III nimmt das resultierende Signal zu.
Abbildung 2: Oxidierte Proteine wirken blutstillend/prothrombotischSteigerung der Thrombozyten-Adhäsion/Inhibition dieser Reaktion
   2a) Oxidierte Proteine steigern die Plättchenadhäsion an Kollagen Typ I. Die Adhäsion der Thrombozyten wurde nach Santoro et al. 1994 durchgeführt. Eine 96-Well-Zellkulturplatte wurde mit Kollagen Typ I (25µg/ml; 100/µl/well) über Nacht bei 4°C gecoatet und die Platten mit BSA geblockt. Humane Thrombozyten wurden durch Gelfiltration in HEPES-Tyrode-Puffer pH7.4 mit Zusatz von 2 mM Mg²⁺, 1mM Mn²⁺, 0.9% Glukose und 0.35% BSA von Plasmaproteinen gereinigt. 100µl gelfiltrierte Plättchen (300000/µl) wurden mit und ohne oxidierten Proteinen für 1 Stunde bei RT in einer feuchten Kammer in den Wells inkubiert. Nicht adhärierte Thrombozyten wurden gründlich ausgewaschen. Die Anzahl der adhärierten Plättchen wurde durch Lyse der Plättchen mit Triton X-100 und Nachweis des lysosomalen Enzyms Hexosaminidase bestimmt. Zur Kalibrierung des Adhäsions-Assays wurde auf der Mikrotiterplatte eine Eichreihe bekannter ansteigender Plättchenanzahl gegeben und die Extinktion des umgesetzten Substrates P-Nitrophenyl-N-Acetyl-β-D-Glucosaminid im Verhältnis zur Plättchenzahl bestimmt. Oxidiertes Antithrombin III steigerte dosisabhängig die Thrombozytenadhäsion.
   2b) Thrombozyten wurden im oben beschriebenen Adhäsions-Assay eingesetzt und mit 50µg/ml oxidiertem ATIII aktiviert. Zusatz von löslichem gereinigtem Thrombospondin inhibierte die durch oxidiertes ATIII gesteigerte Thrombozytenadhäsion dosisabhängig.
   2c) Thrombozyten wurden im oben beschriebenen Adhäsionsassay eingesetzt und mit oxidiertem ATIII aktiviert. Monoklonale Antikörper, die die Bindung von oxidierten Proteinen an CD36 hemmen wie die Klone 37, 13 und 7, hemmen die Wirkung von oxidiertem ATIII. Alle Experimente zum Einfluß von Antikörpern auf Thrombozytenfunktionen wurden in Gegenwart sättigender, komplett blockierender Konzentrationen von Fab-Fragmenten eines Antikörpers gegen den FcRIIA-Rezeptor (Klon IV.3) durchgeführt, um Fc-Rezeptoreffekte auszuschließen.
   2d) Dieser Effekt ist dosisabhängig.
   2e) Die Inhibition der Thrombozytenadhäsion durch lösliches Thrombospondin-1 wird nicht über dessen Bindungsstelle an CD36 direkt (Peptid VTCG) vermittelt. VTCG zeigt keinen Einfluß auf die Steigerung der Thrombozytenadhäsion durch oxidierte Proteine (hier oxidiertes ATIII).
   2f) Antikörper gegen CD36, die die Bindung von CD36 an oxidierte Proteine nicht inhibieren, wie der Klon FA6/152, rufen dagegen keine signifikante Hemmung hervor. Alle Experimente zum Einfluß von Antikörpern auf Thrombozytenfunktionen wurden in Gegenwart sättigender, komplett blockierender Konzentrationen von Fab-Fragmenten eines Antikörpers gegen den FcRIIA-Rezeptor (Klon IV.3) durchgeführt, um Fc-Rezeptoreffekte auszuschließen.

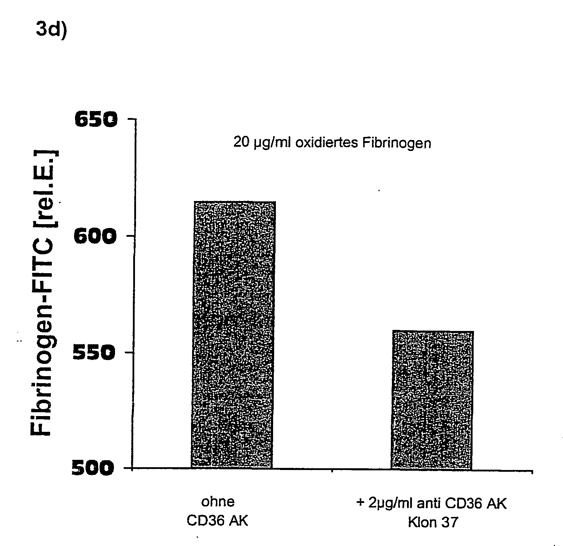

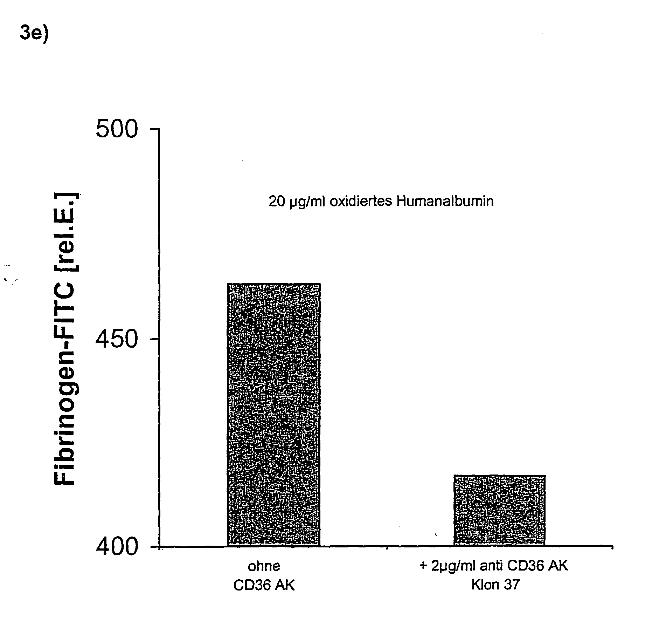

Abbildung 3: Oxidierte Proteine wirken blutstillend/prothrombotischSteigerung der Fibrinogenanbindung an Thrombozyten/Inhibition dieser Reaktion
   Gelfiltrierten Plättchen (50000/µl) in HEPES-Tyrode-BSA-Puffer wurde FITC konjugiertes Fibrinogen und 10µg/ml Thrombospondin zugesetzt. Ein Teil der Proben wurde mit oxidierten Proteinen in aufsteigenden Konzentrationen versetzt. Nach Inkubation für 30 Minuten bei RT wurde die Fibrinogenanbindung im Durchflußzytometer bestimmt.
   3a) Oxidierte Proteine (hier als Beispiel oxidiertes Fibrinogen, oxidiertes Humanalbumin und oxidiertes Antithrombin III) verstärken die Fibrinogenanbindung an Thrombozyten.
   3b) Lösliches Thrombospondin-1 hemmt dosisabhängig die durch ox. Protein ausgelöste Fibrinogenbindung.
   3c) Antikörper, die die Bindung von oxidierten Proteinen an CD36 hemmen, inhibieren dosisabhängig die durch oxidierte Proteine induzierte Fibrinogenanbindung an Thrombozyten oxidiertes Protein: oxidiertes ATIII;
      Antikörper: anti CD36 AK, Klon 37.
   3d) oxidiertes Protein: oxidiertes Fibrinogen;
      Antikörper: anti CD36 AK, Klon 37.
   3e) oxidiertes Protein: oxidiertes Humanalbumin
      Antikörper: anti CD36 AK, Klon 37.
      Alle Experimente zum Einfluß von Antikörpern auf Thrombozytenfunktionen wurden in Gegenwart sättigender, komplett blockierender Konzentrationen von Fab-Fragmenten eines Antikörpers gegen den FcRIIA-Rezeptor (Klon IV.3) durchgeführt, um Fc-Rezeptoreffekte auszuschließen.
Abbildung 4: Oxidierte Proteine wirken blutstillend/prothrombotisch/Induktion der Thrombozytenaggregation/Inhibition dieser Reaktion
   4a) Oxidierte Proteine induzieren die Thrombozytenaggregation. Die Thrombozytenaggregation wurde nach Born 1962 durchgeführt. Zu gelfiltrierten Plättchen (200000/µl) in HEPES-Tyrode Puffer pH 7.4 mit 100µg/ml Fibrinogen wurde in einer Aggregationsküvette TSP-1 (25µg/ml) pipettiert. Lösliches TSP-1 löste alleine keine Aggregation aus. Gleichzeitige Gabe von oxidierten Proteinen (hier oxidiertes Fibrinogen oder oxidiertes Antithrombin III) führte zu einer starken Aggregatbildung. Lösliches Thrombospondin hemmte in hohen Konzentrationen > 50µg/ml die durch oxidierte Proteine ausgelöste Aggregation.
   4b) Antikörper, welche die Bindung von oxidierten Proteinen an CD36 hemmen, inhibieren dosisabhängig die durch oxidierte Proteine induzierte Plättchenaggregation. Alle Experimente zum Einfluß von Antikörpern auf Thrombozytenfunktionen wurden in Gegenwart sättigender, komplett blockierender Konzentrationen von Fab-Fragmenten eines Antikörpers gegen den FcRIIA-Rezeptor (Klon IV.3) durchgeführt, um Fc-Rezeptoreffekte auszuschließen.

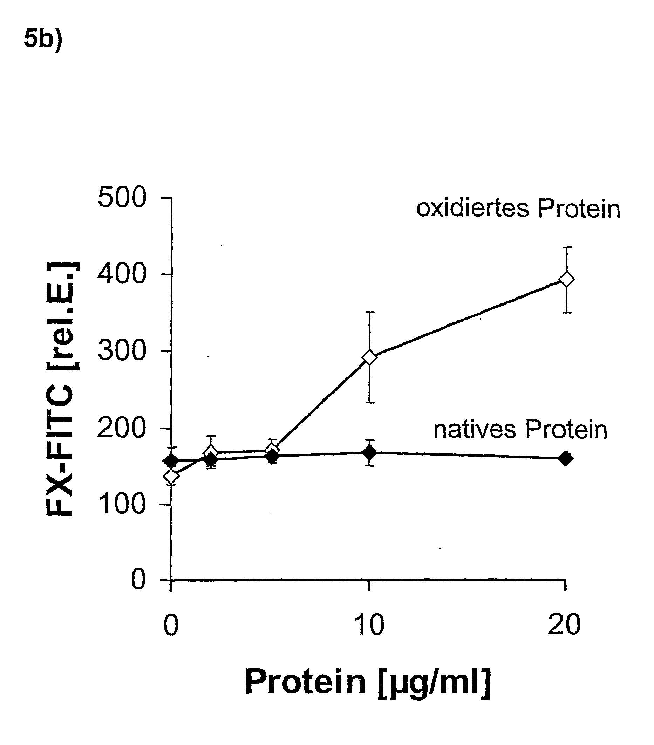

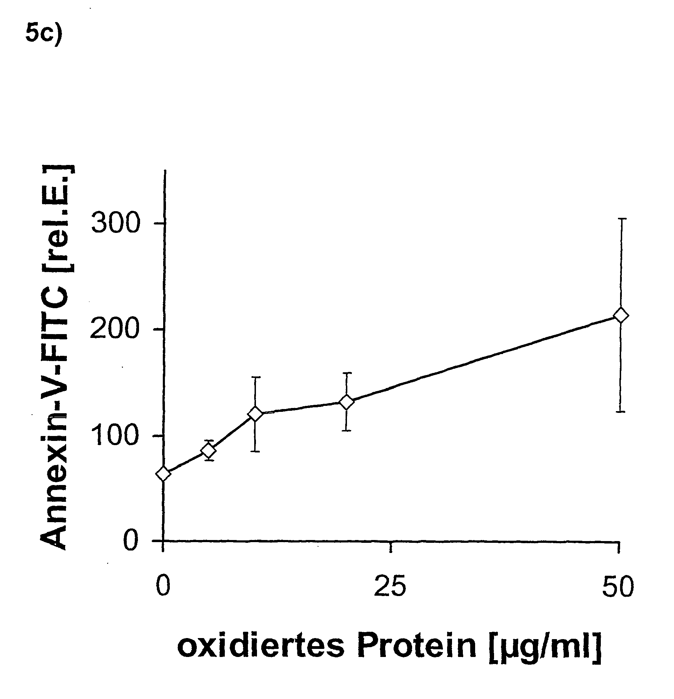

Abbildung 5: Oxidierte Proteine wirken blutstillend/prothrombotischInduktion des prokoagulanten Zustands der Thrombozyten und der Mikropartikelbildung/Inhibition dieser Reaktion
   5a) Oxidierte Proteine (hier als Beispiel oxidiertes Fibrinogen) induzieren eine Bindung von FaktorV/Va an Thrombozyten. Die Faktor V/Va-Bindung wurde wie in Dörmann et al. 1998 beschrieben durchgeführt.
   5b) Oxidierte Proteine (hier als Beispiel oxidiertes Fibrinogen) induzieren die Bindung von FaktorX/Xa an Thrombozyten. Die Faktor X/Xa-Bindung wurde wie in Dörmann et al. 1998 von uns beschrieben durchgeführt.
   5c) Oxidierte Proteine (hier als Beispiel oxidiertes Fibrinogen) induzieren den Phospholipid Flip-Flop in der Membran und damit die Bindung von Annexin V an Thrombozyten. Die Annexin V-Bindung wurde wie in Dörmann et al. 1998 von uns beschrieben durchgeführt.
   5d) Oxidierte Proteine (hier als Beispiel oxidiertes Fibrinogen) induzieren die Mikropartikelbildung von Thrombozyten. Gelfiltrierte Plättchen (50000/µl) wurden mit oxidiertem Protein für30 Minuten bei RT unter leichtem Schwenken inkubiert. Danach wurden die Plättchen und aus Plättchen entstandene Mikropartikel für 30 Minuten mit dem anti GPIX-PE Antikörper inkubiert und die Zahl der entstandenen Mikropartikel im Verhältnis zu 5000 gezählten Partikeln im Durchflußzytometer gemessen.
   5e) Lösliches Thrombospondin hemmt die durch oxidierte Proteine ausgelöste Mikropartikelbildung. In diesem Versuchsbeispiel wurde die Mikropartikelbildung aus Thrombozyten durch oxidiertes Humanalbumin induziert. Gelfiltrierte Plättchen in Hepes-Tyrode Puffer pH 7.4 wurden mit je 50µg/ml oxidiertem Humanalbumin für 1 h bei RT aktiviert. Die Plättchensuspension wurde vor Aktivierung mit löslichem TSP in den angegebenen Konzentrationen versetzt. Die Mikropartikelbildung wurde wie in 5d) beschrieben analysiert. Lösliches Thrombospondin inhibierte dosisabhängig die Bildung der Mikropartikel.
   5f) Anti CD36 Klon37 hemmt die oxProtein induzierte Ausbildung des prokoagulanten Zustands der Plättchen. Die' AnnexinV-Bindung, als Indikator für die Ausbildung einer prokoagulanten Membranoberfläche der Thrombozyten, wurde wie unter 5c) beschrieben gemessen. Vorinkubation der Plättchen mit anti CD36 Antikörpern (30 Minuten, RT), welche die Bindung von ox. Proteinen an CD36 hemmen, inhibierte die nachfolgende Aktivierung dieser Plättchen durch oxidierte Proteine (hier als Beispiel oxidiertes Fibrinogen). Alle Experimente zum Einfluß von Antikörpern auf Thrombozytenfunktionen wurden in Gegenwart sättigender, komplett blockierender Konzentrationen von Fab-Fragmenten eines Antikörpers gegen den FcRIIA-Rezeptor (Klon IV.3) durchgeführt, um Fc-Rezeptoreffekte auszuschließen.
   5g) Anti CD36 AK 37 hemmt die durch oxidierte Proteine ausgelöste Mikropartikelbildung von Thrombozyten. Die Mikropartikelbildung wurde wie unter 5d) beschrieben bestimmt. Vorinkubation der Plättchen mit anti CD36 Antikörpern (30 Minuten, RT), welche die Bindung von oxidierten Proteinen an CD36 hemmen, vor Aktivierung mit oxidierten Proteinen (hier als Beispiel oxidiertes Fibrinogen) hemmt die Mikropartikelbildung.
Abbildung 6: Oxidierte Proteine aktivieren Leukozyten --- Ca²⁺-Signal
   Oxidierte Proteine (hier gezeigt oxidiertes Antithrombin III) induzieren in Monozyten ein Ca²⁺-Signal. Die Ca²⁺ -Messungen wurden nach Sozzani et al. 1993 durchgeführt. Elutriierte Monozyten (5x10⁶/ml) wurden bei RT mit HEPES-Tyrode Puffer pH7.4 gewaschen und anschließend 15 Minuten mit 1 µM Fura2/AM bei 37°C markiert, zweimal mit HEPES-Tyrode Puffer ohne Ca²⁺ gewaschen und dann in HEPES-Tyrode mit 1 mM Ca²⁺ aufgenommen. Ca²⁺-Signal, induziert durch oxidierte Proteine und als Positiv- oder Negativkontrollen wirksame Substanzen, wurden fluorimetrisch im Hitachi F-2000 bestimmt. Oxidiertes Antithrombin III (100µg/ml) aktiviert Monozyten und ruft ein deutliches Ca²⁺-Signal hervor.
Abbildung 7: Oxidierte Proteine aktivieren Leukozyten --- oxidativer Burst
   Oxidierte Proteine verstärken dosisabhängig die aktivierende Wirkung von fMLF auf den oxidativen Burst von PMNL und lösen ihn sogar als selbständige, unabhängige Agonisten auch aus. Die Induktion des oxidativen Burst wurde im wesentlichen gemäß Anleitung des Herstellers mit dem Phagotest/Burst-Test der Firma Orpegen (Heidelberg) am Durchflußzytometer durchgeführt, jedoch zuerst die PMNL mit dem Substrat DHR123 inkubiert und anschließend die PMNL aktiviert. Oxidiertes Antithrombin III steigerte dabei die aktivierende Wirkung von fLMF und rief selbst eine ox. Burst-Reaktion hervor.
Abbildung 8: Oxidierte Proteine aktivieren Leukozyten --- Transmigration durch das Endothel/Inhibition dieser Reaktion
   8a) In "Transwell "-Zellkulturkammern (Costar, Bodenheim) wurde je ein mit einer mikroporösen Polycarbonat-Membran bespannter "Transwell "-Einsatz pro einer der 24 Vertiefungen platziert. Die Polycarbonat-Membran mit einer Porengröße von 5µm wurde mit Fibronectin beschichtet und darauf humane mikrovaskuläre Endothelzellen (HMEC-1) bis zur Konfluenz kultiviert. Durch Dichtegradientenzentrifugation isolierte humane Monozyten (200µl mit 2x10⁷ Zellen/ml in DMEM aus dem periphären Blut) wurden bei 37°C, 7% CO₂ mit dem HMEC-1 Monolayer inkubiert. Als Maß für die Transmigrationsrate wurde die Anzahl der Monozyten im unteren "Transwell"-Kompartiment unter dem "Transwell"-Einsatz bestimmt. Um den Einfluß von verschiedenen oxidierten Proteinen, von Thrombospondin oder von anti CD36 Antikörpern zu untersuchen, wurden die Testsubstanzen dem Medium in der oberen "Transwell "-Kammer zugesetzt oder die Endothelzellen für 10 Minuten mit den Testsubstanzen vorinkubiert und gewaschen. Nach 4-7 Stunden Transmigrationsdauer wurden die Einsätze vorsichtig entfernt, die Zellkulturplatte für 30 Minuten zur Ablösung der adhärierten Monozyten auf Eis gestellt und die Zahl der transmigrierten Monozyten ausgezählt. Oxidiertes Protein (hier oxidiertes ATIII) förderte die Transmigration von Monozyten durch ein HMEC-1 Monolayer, während das nicht oxidierte Ausgangsprotein diese Reaktion nicht zeigte (Transmigrationsdauer 4h).
   8b) Durch Vorinkubation des Endothellayers für 10 Minuten mit TSP-1, bzw. Zusatz von TSP-1 zum Kulturmedium während des Transmigrationsversuchs konnte die Transmigration der Monozyten signifikant gehemmt werden (Transmigrationsdauer 7h).
Abbildung 9: Oxidierte Proteine induzieren Prozesse, welche die Arteriosklerose fördern
   Oxidiertes Antithrombin III steigerte das "Homing" von Makrophagen in arteriosklerotische Plaques. Die Durchführung des Versuchs wurde in der Beschreibung des Beispiels im Detail beschrieben.
Abbildung 10: Thrombospondin hemmt proarteriosklerotische Prozesse
   Thrombospondin hemmt die Adhäsion von Makrophagen an arteriosklerotisch veränderte Endothelzellen. Die Versuchsdurchführung ist in der Beschreibung des Beispiels im Detail erläutert.
   10a) Thrombospondin-1 hemmt die transiente, durch Rollen von Makrophagen gekennzeichnete Adhäsion, an arteriosklerotisch verändertes Endothel.
   10b) Thrombospondin hemmt die permanente, stabile Adhäsion von Makrophagen an arteriosklerotisch verändertes Endothel.

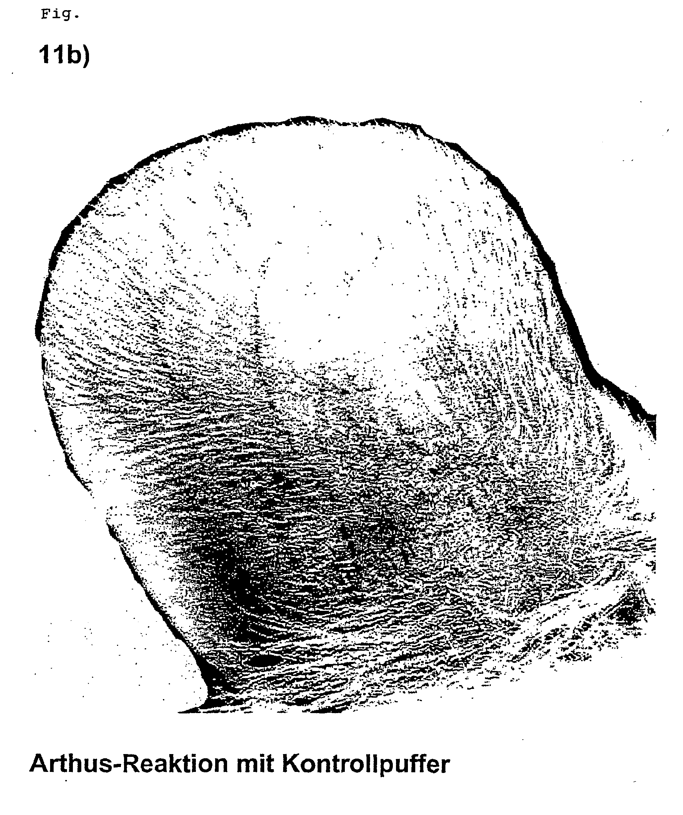

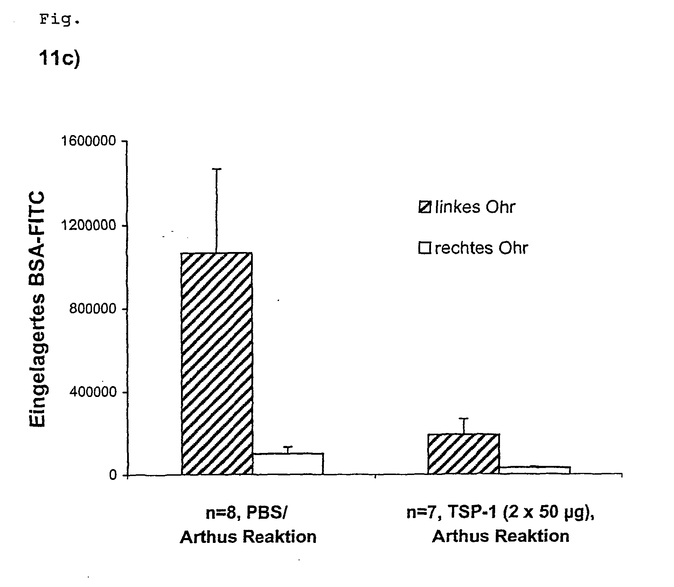

Abbildung 11: Thrombospondin hemmt Entzündungsprozesse in vivo
   Lösliches Thrombospondin-1 inhibiert die Arthus-Reaktion im Ohr von Balb/c Mäusen
   11a) Zweimal zum Zeitpunkt 0 und 0+3 Stunden je 50µg TSP-1 i.p. behandelte Maus --- verhinderte Arthus-Reaktion im linken Ohr
   11b) Zweimal zum Zeitpunkt 0 und 0+3 Stunden mit dem Kontrollpuffer i.p. behandelte Maus --- Arthus-Reaktion im linken Ohr
   11c) In den Ohren eingelagertes BSA-FITC als Maß für die Arthus-Reaktion in mit TSP-1 und Kontrollpuffer behandelten Mäusen --- Arthus-Reaktion im linken Ohr.
Abbildung 12:
   Gelfiltrierte humane Thrombozyten wurden mit Hepes-Tyrode Puffer pH 7.4 auf 50000/µl verdünnt und bei Raumtemperatur aktiviert. Zum Ausschluß von Fc-Rezeptoreffekten auf die Plättchen durch Antikörper wurde das Experiment in Gegenwart sättigender, komplett blockierender Konzentrationen von Fab-Fragmenten eines Antikörpers gegen den FcRIIA-Rezeptor (Klon IV.3) durchgeführt. Nach der Aktivierung wurden die Thrombozyten mit 0,1 % Paraformaldehyd in Hepes-Tyrode Puffer pH 7.4 für 30 Minuten fixiert und gewaschen. Fixierte ruhende und aktivierte Thrombozyten wurden mit anti CD36 "AK36P" bzw. anti CD36 "AK36-gesamt" in sättigenden Konzentrationen über Nacht inkubiert, die Thrombozyten gewaschen und mit einem sekundären, FITC-markierten Antikörper (Ziege anti Kaninchen 1gG-FITC, "minimal X-Reaktion mit human IgG") für 1 h bei RT inkubiert. Die Thrombozyten wurden wiederum gewaschen und die Anbindung der anti CD36 "AK36P" bzw. anti CD36 "AK36-gesamt" Antikörper durch die FITC-Fluoreszenz im Durchflußzytometer (FACScan-Becton Dickinson) quantifiziert (analog Dörmann et al1998).
   Abnahme der Bindung von anti CD36 "AK36P" an Thrombozyten durch Aktivierung.
Abbildung 13
   Patienten mit Diabetes mellitus Typ I und Kontrollprobanden wurde Blut entnommen und dieses mit Citrat antikoaguliert. Durch Zentrifugation wurde Plättchen-reiches Plasma (PRP) hergestellt. Das PRP von Patienten und gesunden Kontrollprobanden wurde mit Fibrinogen (150µg/ml), welches an FITC gekoppelt worden war, versetzt und die Thrombozyten mit oxidiertem Protein (hier oxidiertes Humanalbumin) in aufsteigenden Konzentrationen für 30 Minuten aktiviert. Die Fibrinogenbindung wurde wie oben im Detail beschrieben im Durchflußzytometer gemessen. Die Abbildung zeigt ein charakteristisches Beispiel für die gleichzeitig durchgeführten Aktivierbarkeitsbestimmungen mit PRP von Patienten mit Diabetes mellitus Typ I im Vergleich zu Kontrollen. Thrombozyten von Patienten mit Diabetes mellitus Typ I sind besonders sensitiv für die Aktivierung mit oxidierten Proteinen.
Abbildung 14
   Die Interaktion zwischen oxidierten Proteinen und HIV-Rezeptor CD4 wurde, wie in der Beschreibung des Beispiels im Detail erläutert, mittels Plasmon Resonanz-Technik im BIACORE-System 2000 bestimmt.
   Overlay-Plot von 12 Sensorgrammen, welche die Anbindung von oxidiertem Antithrombin III an immobilisiertes CD4 und die Dissoziation von oxidiertem Antithrombin III zeigen. CD4 ist immobilisiert (148pg); die Konzentration an oxidiertem Antithrombin III wurde variiert (von unten nach oben: OnM; 1 nM; 5.1 nM; 10.2nM; 17nM; 20.4nM; 23nM; 34nM; 40.8nM; 51 nM; 85nM; 119nM). Mit steigender Konzentration an oxidiertem AT III nimmt das resultierende Signal zu.
Abbildung 15
   15a) Oxidiertes Protein vermittelt die TSP-1 Bindung an Thrombozyten Gelfiltrierte humane Thrombozyten wurden mit Hepes-Tyrode Puffer pH 7,4 auf 50000/µl) verdünnt und FITC konjugiertes, gereinigtes Thrombospondin-1 (50µg/ml) zugesetzt. Die Thrombozyten wurden 1 h bei RT mit oxidiertem Protein (hier oxidiertes Fibrinogen) inkubiert und die TSP-1 Bindung an die Thrombozyten im Durchflußzytometer gemessen. Oxidierte Proteine induzieren die TSP-1 Bindung an Thrombozyten.
   15b) Oxidiertes Protein (hier oxidiertes Antithrombin III) induziert die Bindung von Thrombospondin an Endothelzellen.
      Humane, mikrovaskuläre Endothelzellen (HMEC-1) wurden nach Standardverfahren von der Zellkulturplatte abgelöst, vereinzelt und die Suspension für 1 h bei RT mit oxidiertem Protein bzw. oxidiertem Protein plus TSP-1 Zusatz inkubiert. Die Zellen wurden gewaschen und gebundenes TSP-1 mit mAK anti TSP-1 (Klon P10), gekoppelt an Phycoerythrin (PE), markiert und im Durchflußzytometer quantifiziert. Oxidiertes Protein induziert die TSP-1 Bindung an Endothelzellen.
   15c) Während ohne Zusatz von gereinigtem TSP-1 und ohne Zusatz von oxidiertem Antithrombin III nur ca. 1 % der elutriierten Monozyten durch eine Antikörper (Klon P10), der TSP-1 auf der Zelloberfläche erkennt, im Durchflußzytometer detektiert werden, steigt die Anzahl durch Zusatz von gereinigtem TSP-1 (10µg/ml) auf ca. 5%. Zusatz von oxidiertem AT III (ohne Zusatz von exogenem TSP-1) vermittelt die Bindung von endogenem TSP-1 an die Monozyten. Ca. 18% der Monozyten wurden TSP-1 positiv. Durch gleichzeitige Gabe von TSP-1 und oxidiertem AT III wurden nahezu alle der verwendeten periphären Blutmonozyten stark positiv für TSP-1.
   15d) Oxidiertes Protein induziert die Bindung von TSP-1 an T-Zellen. Kultivierte humane T-Zellen (Jurkat-Zellen) wurden für 1 h bei RT mit oxidiertem Protein (hier oxidiertes Antithrombin III) oder oxidiertem Protein plus TSP-1 Zusatz (25µg/ml) inkubiert. An die T-Zellen gebundenes TSP-1 wurde durch den monoklonalen PE-konjugierten anti TSP Antikörper (Klon P10) markiert und im Durchflußzytometer gemessen. Oxidierte Proteine induzieren die Bindung von endogen vorhandenem und exogen zugesetztem TSP an T-Zellen.
Abbildung 16
   Diese Abbildung verdeutlicht den Mechanismus entsprechend der Erfindung.

### Reference List

1. Abumrad N, Coburn C, Ibrahimi A: Membrane proteins implicated in long-chain fatty acid uptake by mammalian cells: CD36, FATP and FABPm. Biochim.Biophys.Acta 1441: 4-13, 1999
2. Abumrad NA, el Maghrabi MR, Amri EZ, Lopez E, Grimaldi PA: Cloning of a rat adipocyte membrane protein implicated in binding or transport of long-chain fatty acids that is induced during preadipocyte differentiation. Homology with human CD36. J.Biol.Chem. 268: 17665-17668, 1993
3. Acton SL, Scherer PE, Lodish HF, Krieger M: Expression cloning of SR-BI, a CD36-related class B scavenger receptor. J.Biol.Chem. 269: 21003-21009, 1994
4. Aiken ML, Ginsberg MH, Byers-Ward V, Plow EF: Effects of OKM5, a monoclonal antibody to glycoprotein IV, on platelet aggregation and thrombospondin surface expression [see comments]. Blood 76: 2501-2509, 1990
5. Alberts GL, Pregenzer JF, Im WB: Contributions of cysteine 114 of the human D3 dopamine receptor to ligand binding and sensitivity to external oxidizing agents. Br.J.Pharmacol. 125: 705-710, 1998
6. Alessio M, Roggero S, Bussolino F, Saitta M, Malavasi F: Characterization of the murine monoclonal antibody NL07 specific for the human thrombospondin receptor (CD36 molecule). Curr.Stud.Hematol.Blood Transfus. 182-186, 1991
7. Alessio M, Greco NJ, Primo L, Ghigo D, Bosia A, Tandon NN, Ockenhouse CF, Jamieson GA, Malavasi F: Platelet activation and inhibition of malarial cytoadherence by the anti-CD36 lgM monoclonal antibody NL07. Blood 82: 3637-3647, 1993
8. Asch AS, Barnwell J, Silverstein RL, Nachman RL: Isolation of the thrombospondin membrane receptor. J.Clin.lnvest 79: 1054-1061, 1987
9. Asch AS, Nachman RL: Thrombospondin: phenomenology to function. Prog.Hemost.Thromb. 9: 157-176, 1989
10. Asch AS, Liu I, Briccetti FM, Barnwell JW, Kwakye-Berko F, Dokun A, Goldberger J, Pernambuco M: Analysis of CD36 binding domains: ligand specificity controlled by dephosphorylation of an ectodomain. Science 262: 1436-1440, 1993
11. Babior BM: Oxygen-dependent microbial killing by phagocytes (second of two parts). N.Engl.J.Med. 298: 721-725, 1978
12. Babior BM: Oxygen-dependent microbial killing by phagocytes (first of two parts). N.Engl.J.Med. 298: 659-668, 1978
13. Barnwell JW, Asch AS, Nachman RL, Yamaya M, Aikawa M, Ingravallo P: A human 88-kD membrane glycoprotein (CD36) functions in vitro as a receptor for a cytoadherence ligand on Plasmodium falciparum-infected erythrocytes. J.Clin.lnvest 84: 765-772, 1989
14. Baruch DI, Ma XC, Pasloske B, Howard RJ , Miller LH: CD36 peptides that block cytoadherence define the CD36 binding region for Plasmodium falciparum-infected erythrocytes. Blood 94: 2121-2127, 1999
15. Berendt AR, Ferguson DJ, Gardner J, Turner G, Rowe A, McCormick C, Roberts D, Craig A, Pinches R, Elford BC: Molecular mechanisms of sequestration in malaria. Parasitology 108 Suppl: S19-S28, 1994
16. Bosmans JL, Holvoet P, Dauwe SE, Ysebaert DK, Chapelle T, Jurgens A, Kovacic V, Van Marck EA, De Broe ME, Verpooten GA: Oxidative modification of low-density lipoproteins and the outcome of renal allografts at 1 1/2 years. Kidney lnt. 59: 2346-2356, 2001
17. Boullier A, Gillotte KL, Horkko S, Green SR, Friedman P, Dennis EA, Witztum JL, Steinberg D, Quehenberger O: The binding of oxidized low density lipoprotein to mouse CD36 is mediated in part by oxidized phospholipids that are associated with both the lipid and protein moieties of the lipoprotein. J.Biol.Chem. 275: 9163-9169, 2000
18. Brown MS, Goldstein JL: A receptor-mediated pathway for cholesterol homeostasis. Science 232: 34-47, 1986
19. Calvo D, Vega MA: Identification, primary structure, and distribution of CLA-1, a novel member of the CD36/LIMPII gene family. J.Biol.Chem. 268: 18929-18935, 1993
20. Carr AC, McCall MR, Frei B: Oxidation of LDL by myeloperoxidase and reactive nitrogen species: reaction pathways and antioxidant protection. Arterioscler.Thromb.Vasc.Biol. 20: 1716-1723, 2000
21. Clemetson KJ: Biochemistry of platelet membrane glycoproteins. Prog.Clin.Biol.Res. 283: 33-75, 1988
22. Crombie R, Silverstein RL, MacLow C, Pearce SFA, Nachman RL, Laurence J: Identification of a CD36-related thrombospondin 1-binding domain in HIV- 1 envelope glycoprotein gp120: relationship to HIV-1-specific inhibitory factors in human saliva. J.Exp.Med. 187: 25-35, 1998
23. Daniel JL, Dangelmaier C, Strouse R, Smith JB: Collagen induces normal signal transduction in platelets deficient in CD36 (platelet glycoprotein IV). Thromb.Haemost. 71: 353-356, 1994
24. Davies JM, Horwitz DA, Davies KJ: Potential roles of hypochlorous acid and N-chloroamines in collagen breakdown by phagocytic cells in synovitis. Free Radic.Biol.Med. 15: 637-643, 1993
25. Dawson DW, Pearce SF, Zhong R, Silverstein RL, Frazier WA, Bouck NP: CD36 mediates the In vitro inhibitory effects of thrombospondin-1 on endothelial cells. J.Cell Biol. 138: 707-717, 1997
26. Dörmann D, Kardoeus J, Zimmermann RE, Kehrel B: Flow cytometric analysis of agonist-induced annexin V, factor Va and factor Xa binding to human platelets. Platelets 9: 171-177, 1998
27. Dutta-Roy AK, Crosbie LC, Gordon MJ, Campbell FM: Platelet membrane glycoprotein IV (CD36) is involved in arachidonic acid induced-platelet aggregation. Biochem.Soc.Trans. 24: 167S, 1996
28. Fadok VA, Warner ML, Bratton DL, Henson PM: CD36 is required for phagocytosis of apoptotic cells by human macrophages that use either a phosphatidylserine receptor or the vitronectin receptor (alpha v beta 3). J.Immunol. 161: 6250-6257, 1998
29. Fadok VA, Bratton DL, Konowal A, Freed PW, Westcott JY, Henson PM: Macrophages that have ingested apoptotic cells in vitro inhibit proinflammatory cytokine production through autocrine/paracrine mechanisms involving TGF-beta, PGE2, and PAF. J.Clin.Invest 101: 890-898, 1998
30. Febbraio M, Abumrad NA, Hajjar DP, Sharma K, Cheng W, Pearce SF, Silverstein RL: A null mutation in murine CD36 reveals an important role in fatty acid and lipoprotein metabolism. J.Biol.Chem. 274: 19055-19062, 1999
31. Febbraio M, Podrez EA, Smith JD, Hajjar DP, Hazen SL, Hoff HF, Sharma K, Silverstein RL: Targeted disruption of the class B scavenger receptor CD36 protects against atherosclerotic lesion development in mice [see comments]. J.Clin.lnvest 105: 1049-1056, 2000
32. Frieda S, Pearce A, Wu J, Silverstein RL : Recombinant GST/CD36 fusion proteins define a thrombospondin binding domain. Evidence for a single calcium-dependent binding site on CD36. J.Biol.Chem. 270: 2981-2986, 1995
33. Glaser CB, Morser J, Clarke JH, Blasko E , McLean K, Kuhn I, Chang RJ, Lin JH, Vilander L, Andrews WH, .: Oxidation of a specific methionine in thrombomodulin by activated neutrophil products blocks cofactor activity. A potential rapid mechanism for modulation of coagulation. J.Clin.lnvest 90: 2565-2573, 1992
34. Greenwalt DE, Watt KW, So OY, Jiwani N: PAS IV, an integral membrane protein of mammary epithelial cells, is related to platelet and endothelial cell CD36 (GP IV). Biochemistry 29: 7054-7059, 1990
35. Greenwalt DE, Lipsky RH, Ockenhouse CF, Ikeda H, Tandon NN, Jamieson GA: Membrane glycoprotein CD36: a review of its roles in adherence, signal transduction, and transfusion medicine. Blood 80: 1105-1115, 1992
36. Hansson M, Asea A, Ersson U, Hermodsson S, Hellstrand K: Induction of apoptosis in NK cells by monocyte-derived reactive oxygen metabolites. J.Immunol. 156: 42-47, 1996
37. Hazell LJ, Arnold L, Flowers D, Waeg G, Malle E, Stocker R: Presence of hypochlorite-modified proteins in human atherosclerotic lesions. J.Clin.lnvest 97: 1535-1544, 1996
38. Holvoet P, Collen D: Oxidized lipoproteins in atherosclerosis and thrombosis. FASEB J. 8: 1279-1284, 1994
39. Holvoet P, Collen D: Oxidation of low density lipoproteins in the pathogenesis of atherosclerosis. Atherosclerosis 137 Suppl: S33-S38, 1998
40. Holvoet P, Stassen JM, Van Cleemput J, Collen D, Vanhaecke J: Oxidized low density lipoproteins in patients with transplantassociated coronary artery disease. Arterioscler.Thromb.Vasc.Biol. 18: 100-107, 1998
41. Holvoet P, Van Cleemput J, Collen D, Vanhaecke J: Oxidized low density lipoprotein is a prognostic marker of transplant- associated coronary artery disease. Arterioscler.Thromb.Vasc.Biol. 20: 698-702, 2000
42. Holvoet P, Mertens A, Verhamme P, Bogaerts K, Beyens G, Verhaeghe R, Collen D, Muls E, Van de WF: Circulating oxidized LDL is a useful marker for identifying patients with coronary artery disease. Arterioscler.Thromb.Vasc.Biol. 21: 844-848, 2001
43. Huang MM, Bolen JB, Barnwell, JW, Shattil SJ, Brugge JS: Membrane glycoprotein IV (CD36) is physically associated with the Fyn, Lyn, and Yes protein-tyrosine kinases in human platelets. Proc.Natl.Acad.Sci.U.S.A 88: 7844-7848, 1991
44. Jimenez B, Volpert OV, Crawford SE, Febbraio M, Silverstein RL, Bouck N: Signals leading to apoptosis-dependent inhibition of neovascularization by thrombospondin-1. Nat.Med. 6: 41-48, 2000
45. Kehrel B, Kronenberg A, Schwippert B, Niesing-Bresch D, Niehues U, Tschope D, van de LJ, Clemetson KJ: Thrombospondin binds normally to glycoprotein IIIb deficient platelets. Biochem.Biophys.Res.Commun. 179: 985-991, 1991
46. Kehrel B, Kronenberg A, Rauterberg J, Niesing-Bresch D, Niehues U, Kardoeus J, Schwippert B, Tschope D, van de LJ, Clemetson KJ: Platelets deficient in glycoprotein IIIb aggregate normally to collagens type I and III but not to collagen type V. Blood 82: 3364-3370, 1993
47. Kehrel B: Platelet-collagen interactions. Semin.Thromb.Hemost. 21: 123-129, 1995
48. Kieffer N, Bettaieb A, Legrand C, Coulombel L, Vainchenker W, Edelman L, Breton-Gorius J: Developmentally regulated expression of a 78 kDa erythroblast membrane glycoprotein immunologically related to the platelet thrombospondin receptor. Biochem.J. 262: 835-842, 1989
49. Kielbassa K, Schmitz C, Gerke V: Disruption of endothelial microfilaments selectively reduces the transendothelial migration of monocytes. Exp.Cell Res. 243: 129-141, 1998
50. Knowles DM, Tolidjian B, Marboe C, D'Agati V, Grimes M, Chess L: Monoclonal anti-human monocyte antibodies OKM1 and OKM5 possess distinctive tissue distributions including differential reactivity with vascular endothelium. J.lmmunol. 132: 2170-2173, 1984
51. Kronenberg A, Grahl H, Kehrel B: Human platelet CD36 (GPIIIb, GPIV) binds to cholesteryl-hemisuccinate and can be purified by a simple two-step method making use of this property. Thromb.Haemost. 79: 1021-1024, 1998
52. Leung LL, Li WX, McGregor JL, Albrecht G, Howard RJ: CD36 peptides enhance or inhibit CD36-thrombospondin binding. A two-step process of ligand-receptor interaction. J.Biol.Chem. 267: 18244-18250, 1992
53. Li WX, Howard RJ, Leung LL: Identification of SVTCG in thrombospondin as the conformation- dependent, high affnity binding site for its receptor, CD36. J.Biol.Chem. 268: 16179-16184, 1993
54. Lian EC, Siddiqui FA, Jamieson GA, Tandon NN: Platelet agglutinating protein p37 causes platelet agglutination through its binding to membrane glycoprotein IV. Thromb.Haemost. 65: 102-106, 1991
55. McGregor JL, Clemetson KJ, James E, Luscher EF, Dechavannne M: Characterization of human blood platelet membrane proteins and glycorproteins by their isoelectric point (pl) and apparent molecular weight using two-dimensional electrophoresis and surface-labelling techniques. Biochim.Biophys.Acta 599: 473-483, 1980
56. McGregor JL, Clemetson KJ, James E, Capitanio A, Greenland T, Luscher EF, Dechavanne M: Glycoproteins of platelet membranes from Glanzmann's thrombasthenia. A comparison with normal using carbohydrate-specific or protein-specific labelling techniques and highresolution two-dimensional gel electrophoresis. Eur.J.Biochem. 116: 379-388, 1981
57. Nakata A, Nakagawa Y, Nishida M, Nozaki S, Miyagawa J, Nakagawa T, Tamura R, Matsumoto K, Kameda-Takemura K, Yamashita S, Matsuzawa Y: CD36, a novel receptor for oxidized low-density lipoproteins, is highly expressed on lipid-laden macrophages in human atherosclerotic aorta. Arterioscler.Thromb.Vasc.Biol. 19: 1333-1339, 1999
58. Nguyen-Khoa T, Massy ZA, Witko-Sarsat V, Canteloup S, Kebede M, Lacour B , Drueke T, Descamps-Latscha B: Oxidized low-density lipoprotein induces macrophage respiratory burst via its protein moiety: A novel pathway in atherogenesis" Biochem.Biophys.Res.Commun. 263: 804-809, 1999
59. Nicholson AC, Frieda S, Pearce A, Silverstein RL: Oxidized LDL binds to CD36 on human monocyte-derived macrophages and transfected cell lines. Evidence implicating the lipid moiety of the lipoprotein as the binding site. Arterioscler.Thromb.Vasc.Biol. 15: 269-275, 1995
60. Nozaki S, Kashiwagi H, Yamashita S, Nakagawa T, Kostner B, Tomiyama Y , Nakata A, Ishigami M, Miyagawa J, Kameda-Takemura K: Reduced uptake of oxidized low density lipoproteins in monocyte-derived macrophages from CD36-deficient subjects. J.Clin.lnvest 96: 1859-1865, 1995
61. Nozaki S, Tanaka T, Yamashita S, Sohmiya K, Yoshizumi T, Okamoto F, Kitaura Y, Kotake C, Nishida H, Nakata A, Nakagawa T, Matsumoto K, Kameda-Takemura K, Tadokoro S, Kurata Y, Tomiyama Y, Kawamura K, Matsuzawa Y: CD36 mediates long-chain fatty acid transport in human myocardium: complete myocardial accumulation defect of radiolabeled long-chain fatty acid analog in subjects with CD36 deficiency. Mol.Cell Biochem. 192: 129-135, 1999
62. Ockenhouse CF, Tandon NN, Magowan C, Jamieson GA, Chulay JD: Identification of a platelet membrane glycoprotein as a falciparum malaria sequestration receptor [see comments]. Science 243: 1469-1471, 1989
63. Oquendo P, Hundt E, Lawler J, Seed B: CD36 directly mediates cytoadherence of Plasmodium falciparum parasitized erythrocytes. Cell 58: 95-101, 1989
64. Patel SS, Thiagarajan R, Willerson JT, Yeh ET: Inhibition of alpha4 integrin and ICAM-1 markedly attenuate macrophage homing to atherosclerotic plaques in ApoE-deficient mice. Circulation 97: 75-81, 1998
65. Pearce SF, Roy P, Nicholson AC, Hajjar DP, Febbraio M, Silverstein RL: Recombinant glutathione S-transferase/CD36 fusion proteins define an oxidized low density lipoprotein-binding domain. J.Biol.Chem. 273: 34875-34881, 1998
66. Podrez EA, Febbraio M, Sheibani N, Schmitt D, Silverstein RL, Hajjar DP , Cohen PA, Frazier WA, Hoff HF, Hazen SL: Macrophage scavenger receptor CD36 is the major receptor for LDL modified by monocyte-generated reactive nitrogen species [see comments] [published erratum appears in J Clin Invest 2000 May;105(10):1483]. J.Clin.lnvest 105: 1095-1108, 2000
67. Puente N, Daviet L, Ninio E, McGregor JL : Identification on human CD36 of a domain (155-183) implicated in binding oxidized low density lipoproteins (Ox-LDL). Arterioscler.Thromb.Vasc.Biol. 16:1033-1039, 1996
68. Ricciarelli R, Zingg JM, Azzi A: Vitamin E reduces the uptake of oxidized LDL by inhibiting CD36 scavenger receptor expression in cultured aortic smooth muscle cells. Circulation 102: 82-87, 2000
69. Rigotti A, Acton SL, Krieger M: The class B scavenger receptors SR-BI and CD36 are receptors for anionic phospholipids. J.Biol.Chem. 270: 16221-16224, 1995
70. Roberts DD, Sherwood JA, Spitalnik SL, Panton LJ, Howard RJ, Dixit VM, Frazier WA, Miller LH, Ginsburg V: Thrombospondin binds falciparum malaria parasitized erythrocytes and may mediate cytoadherence. Nature 318: 64-66, 1985
71. Ryeom SW, Sparrow JR, Silverstein RL: CD36 participates in the phagocytosis of rod outer segments by retinal pigment epithelium. J.Cell Sci. 109 (Pt 2):.387-395, 1996
72. Saelman EU, Kehrel B, Hese KM, de Groot PG, Sixma JJ, Nieuwenhuis HK: Platelet adhesion to collagen and endothelial cell matrix under flow conditions is not dependent on platelet glycoprotein IV. Blood 83: 3240-3244, 1994
73. Samanta A, Das DK, Jones R, George A, Prasad MR: Free radical scavenging by myocardial fatty acid binding protein. Free Radic.Res.Commun. 7: 73-82, 1989
74. Schraufstatter IU, Browne K, Harris A, Hyslop PA, Jackson JH, Quehenberger O, Cochrane CG: Mechanisms of hypochlorite injury of target cells. J.Clin.Invest 85: 554-562, 1990
75. Schuepp BJ, Pfister H, Clemetson KJ, Silverstein RL, Jungi TW: CD36-mediated signal transduction in human monocytes by anti-CD36 antibodies but not by anti-thrombospondin antibodies recognizing cell membrane-bound thrombospondin. Biochem.Biophys.Res.Commun. 175: 263-270, 1991
76. Shah MM, Aust SD: Oxidation of halides by peroxidases and their subsequent reductions: Arch.Biochem.Biophys. 300: 253-257, 1993
77. Shattil SJ, Brugge JS: Protein tyrosine phosphorylation and the adhesive functions of platelets. Curr.Opin.Cell Biol. 3: 869-879, 1991
78. Silverstein RL, Baird M, Lo SK, Yesner LM: Sense and antisense cDNA transfection of CD36 (glycoprotein IV) in melanoma cells. Role of CD36 as a thrombospondine receptor. J.Biol.Chem. 267: 16607-16612, 1992
79. Smith MA, Rottkamp CA, Nunomura A, Raina AK, Perry G: Oxidative stress in Alzheimer's disease. Biochim.Biophys.Acta 1502: 139-144, 2000
80. Stadtman ER: Protein oxidation and aging. Science 257: 1220-1224, 1992
81. Steinberg D: Low density lipoprotein oxidation and its pathobiological significance. J.Biol.Chem. 272: 20963-20966, 1997
82. Tandon NN, Kralisz U, Jamieson GA: Identification of glycoprotein IV (CD36) as a primary receptor for platelet-collagen adhesion. J.Biol.Chem. 264: 7576-7583, .1989
83. Tandon NN, Ockenhouse CF, Greco NJ, Jamieson GA: Adhesive functions of platelets lacking glycoprotein IV (CD36). Blood 78: 2809-2813,1991
84. Vega MA, Segui-Real B, Garcia JA, Cales C, Rodriguez F, Vanderkerckhove J, Sandoval IV: Cloning, sequencing, and expression of a cDNA encoding rat LIMP 11, a novel 74-kDa lysosomal membrane protein related to the surface adhesion protein CD36. J.Biol.Chem. 266: 16818-16824, 1991
85. Volf 1, Bielek E, Moeslinger T, Koller F, Koller E: Modification of protein moiety of human low density lipoprotein by hypochlorite generates strong platelet agonist. Arterioscler.Thromb.Vasc.Biol. 20: 2011-2018, 2000
86. Weiss SJ: Tissue destruction by neutrophils. N.Engl.J.Med. 320: 365-376, 1989
87. Witztum JL, Steinberg D: Role of oxidized low density lipoprotein in atherogenesis. J.Clin.Invest 88: 1785-1792, 1991
88. Yamaguchi A, Yamamoto N, Akamatsu N, Saido TC, Kaneda M, Umeda M, Tanoue K: PS-liposome and ox-LDL bind to different sites of the immunodominant domain (#155-183) of CD36: a study with GS95, a new anti-CD36 monoclonal antibody. Thromb.Res. 97: 317-326, 2000

## Patentansprüche

1. Arzneimittel zur Verwendung in der Prophylaxe von Thrombosen, insbesondere bei entzündlichen Erkrankungen, enthaltend lösliches Thrombospondin oder Antikörper; die die Bindung von oxidierten Proteinen an CD36 hemmen.

2. Arzneimittel zur Verwendung in der Unterstützung einer anti-thrombotischen Therapie, enthaltend lösliches Thrombospondin oder Antikörper, die die Bindung von oxidierten Proteinen an CD36 hemmen.

3. Arzneimittel zur Verwendung in der Verhinderung einer Trarisplantatabstoßung, enthaltend lösliches Thrombospondin oder Antikörper, die die Bindung von oxidierten Proteinen an CD36 hemmen.

4. Arzneimittel zur Verwendung in der Verhinderung einer transplantationsbedingten Arteriosklerose, enthaltend lösliches Thrombospondin oder Antikörper, die die Bindung von oxidierten Proteinen an CD36 hemmen.

5. Arzneimittel zur Verwendung in der Verhinderung der Entwicklung und des Fortschreitens von arteriosklerotischen (atherosklerotischen) Erkrankungen, enthaltend lösliches Thrombospondin oder Antikörper, die die Bindung von oxidierten Proteinen an CD36 hemmen.

6. Arzneimittel zur Verwendung in der Behandlung von chronischen Entzündungsreaktionen, enthaltend lösliches Thrombospondin oder Antikörper, die die Bindung von oxidierten Proteinen an CD36 hemmen.

7. Arzneimittel zur Verwendung in der Verhinderung einer frühen Gefäß-Reocclusion nach Bypass-Operation, Stent, PTCA oder ähnlichem, enthaltend lösliches Thrombospondin oder Antikörper, die die Bindung von oxidierten Proteinen an CD36 hemmen.

8. Arzneimittel zur Verwendung in der Verhinderung einer Gefäß-Restenose nach Bypass-Operation, Stent, PTCA oder ähnlichem, enthaltend lösliches Thrombospondin oder Antikörper, die die Bindung von oxidierten Proteinen an CD36 hemmen.

9. Arzneimittel zur Verwendung in der Verhinderung von Reperfusionsschäden, enthaltend lösliches Thrombospondin oder Antikörper, die die Bindung von oxidierten Proteinen an CD36 hemmen.

10. Arzneimittel nach Anspruch 9, wobei die Reperfusionschäden im Zusammenhang mit Myokardischämie, Organtransplantation, Schlaganfall, peripherer Verschlusskrankheit, operativen Eingriffen, Multiorganversagen nach erfolgreicher Wiederbelebung stehen.

11. Arzneimittel zur Verwendung in der Verhinderung von Gefäßschädigungen, insbesondere bei Patienten mit Diabetes mellitus, enthaltend lösliches Thrombospondin oder Antikörper, die die Bindung von oxidierten Proteinen an CD36 hemmen.

12. Arzneimittel zur Verwendung in der Unterstützung der Wundheilung, enthaltend lösliches Thrombospondin oder Antikörper, die die Bindung von oxidierten Proteinen an CD36 hemmen.

13. Arzneimittel nach einem der Ansprüche 1 bis 12, **dadurch gekennzeichnet, dass** es monoklonale Antikörper oder Fragmente (F(ab)₂, F(ab), Antigen-Erkennungsregion) derselben enthält.

14. Arzneimittel nach einem der vorhergehenden Ansprüche, **dadurch gekennzeichnet, dass** es weitere pharmazeutisch akzeptable Hilfs-und/oder Trägersubstanzen enthält.

15. Arzneimittel nach einem der vorhergehenden Ansprüche, **dadurch gekennzeichnet, dass** es zur lokalen, intradermalen, oberflächlichen, intravenösen, oralen oder intramuskulären Verabreichung oder zur Verabreichung über Vesikel formuliert ist.

16. Arzneimittel nach einem der vorhergehenden Ansprüche, **dadurch gekennzeichnet, dass** es weitere Substanzen wie z.B. Antibiotika, Immunsuppressiva oder Interaktionspartner von oxidierten Proteinen im Körper enthält.

17. Verfahren zur Qualitätskontrolle von Blutprodukten durch Quantifizierung von oxidierten Proteinen in diesen Blutprodukten, **dadurch gekennzeichnet, dass** die Menge oxidierter Proteine mithilfe von monoklonalen Antikörpern, welche oxidiertes Protein binden, jedoch nicht mit nicht-oxidiertem Protein reagieren, festgestellt wird.

18. Verfahren nach Anspruch 17, **dadurch gekennzeichnet, dass** die monoklonalen Antikörper gegen eines von OxAntithrombin III, OxHumanalbumin oder OxFibrinogen oder diese Proteine insgesamt gerichtet sind.

19. Verfahren nach Anspruch 17 oder 18, **dadurch gekennzeichnet, dass** die monoklonalen Antikörper zur Quantifizierung in einem ELISA- oder RIA-Test oder in einem Verfahren zur quantitativen Durchflusszytometrie auf Zelloberflächen eingesetzt werden.

## Claims

1. A medicament for use in the prophylactic treatment of thrombosis, in particular in inflammatory diseases, containing soluble thrombospondin or antibodies which inhibit the binding of oxidised proteins to CD36.

2. A medicament for use in assisting an antithrombotic therapy, containing soluble thrombospondin or antibodies which inhibit the binding of oxidised proteins to CD36.

3. A medicament for use in preventing transplant rejection, containing soluble thrombospondin or antibodies which inhibit the binding of oxidised proteins to CD36.

4. A medicament for use in preventing transplant-related arteriosclerosis, containing soluble thrombospondin or antibodies which inhibit the binding of oxidised proteins to CD36.

5. A medicament for use in preventing the development and progression of arteriosclerotic (atherosclerotic) diseases, containing soluble thrombospondin or antibodies which inhibit the binding of oxidised proteins to CD36.

6. A medicament for use in treating chronic inflammatory responses, containing soluble thrombospondin or antibodies which inhibit the binding of oxidised proteins to CD36.

7. A medicament for use in preventing early vascular reocclusion following a bypass operation, stent, PTCA or the like, containing soluble thrombospondin or antibodies which inhibit the binding of oxidised proteins to CD36.

8. A medicament for use in preventing vascular restenosis following a bypass operation, stent, PTCA or the like, containing soluble thrombospondin or antibodies which inhibit the binding of oxidised proteins to CD36.

9. A medicament for use in preventing reperfusion injury, containing soluble thrombospondin or antibodies which inhibit the binding of oxidised proteins to CD36.

10. A medicament according to claim 9, wherein the reperfusion injury is associated with myocardial ischaemia, organ transplant, stroke, peripheral occlusive disease, surgical intervention, multiple organ failure after successful resuscitation.

11. A medicament for use in preventing vascular damage, in particular in patients with diabetes mellitus, containing soluble thrombospondin or antibodies which inhibit the binding of oxidised proteins to CD36.

12. A medicament for use in assisting wound healing, containing soluble thrombospondin or antibodies which inhibit the binding of oxidised proteins to CD36.

13. A medicament according to any one of claims 1 to 12, **characterised in that** it contains monoclonal antibodies or fragments (F(ab)₂, F(ab), antigen recognition region) thereof.

14. A medicament according to any one of the preceding claims, **characterised in that** contains further pharmaceutically acceptable auxiliary substances and/or excipients.

15. A medicament according to any one of the preceding claims, **characterised in that** it is formulated for local, intradermal, superficial, intravenous, oral or intramuscular administration or for administration via vesicles.

16. A medicament according to any one of the preceding claims, **characterised in that** it contains further substances such as for example antibiotics, immunosuppressants or interaction partners of oxidised proteins in the body.

17. A method for the quality control of blood products by quantifying oxidised proteins in these blood products, **characterised in that** the quantity of oxidised proteins is established with the assistance of monoclonal antibodies which bind oxidised protein, but which do not however react with unoxidised protein.

18. A method according to claim 17, **characterised in that** the monoclonal antibodies are directed against one of ox-antithrombin III, ox-human albumin or ox-fibrinogen or all of these proteins.

19. A method according to claim 17 or claim 18, **characterised in that** the monoclonal antibodies are used for quantification in an ELISA or RIA test or in a method for quantitative flow cytometry on cell surfaces.

## Revendications

1. Médicament destiné à être utilisé dans la prophylaxie des thromboses, en particulier dans le cas de maladies inflammatoires, contenant de la thrombospondine soluble ou des anticorps qui inhibent la liaison de protéines oxydées au CD36.

2. Médicament destiné à être utilisé pour soutenir un traitement antithrombotique, contenant de la thrombospondine soluble ou des anticorps qui inhibent la liaison de protéines oxydées au CD36.

3. Médicament destiné à être utilisé pour prévenir le rejet d'une greffe, contenant de la thrombospondine soluble ou des anticorps qui inhibent la liaison de protéines oxydées au CD36.

4. Médicament destiné à être utilisé pour prévenir l'artériosclérose après transplantation, contenant de la thrombospondine soluble ou des anticorps qui inhibent la liaison de protéines oxydées au CD36.

5. Médicament destiné à être utilisé pour prévenir le développement et la progression de maladies artériosclérotiques (athérosclérotiques), contenant de la thrombospondine soluble ou des anticorps qui inhibent la liaison de protéines oxydées au CD36.

6. Médicament destiné à être utilisé pour traiter les réactions inflammatoires chroniques, contenant de la thrombospondine soluble ou des anticorps qui inhibent la liaison de protéines oxydées au CD36.

7. Médicament destiné à être utilisé pour prévenir une réocclusion précoce des vaisseaux après une intervention de pontage, de pose d'endoprothèse, d'angioplastie coronarienne transluminale percutanée ou similaire, contenant de la thrombospondine soluble ou des anticorps qui inhibent la liaison de protéines oxydées au CD36.

8. Médicament destiné à être utilisé pour prévenir une resténose des vaisseaux après une intervention de pontage, de pose d'endoprothèse, d'angioplastie coronarienne transluminale.percutanée ou similaire, contenant de la thrombospondine soluble ou des anticorps qui inhibent la liaison de protéines oxydées au CD36.

9. Médicament destiné à être utilisé pour prévenir les lésions de reperfusion, contenant de la thrombospondine soluble ou des anticorps qui inhibent la liaison de protéines oxydées au CD36.

10. Médicament selon la revendication 9, les lésions de reperfusion étant liées à une ischémie myocardique, une transplantation d'organe, une attaque cérébrale, une maladie vasculaire périphérique, une intervention chirurgicale, une défaillance multi-organes après une réanimation réussie.

11. Médicament destiné à être utilisé pour prévenir les lésions vasculaires, en particulier chez les patients atteints de diabète sucré, contenant de la thrombospondine soluble ou des anticorps qui inhibent la liaison de protéines oxydées au CD36.

12. Médicament destiné à être utilisé pour aider à la cicatrisation des plaies, contenant de la thrombospondine soluble ou des anticorps qui inhibent la liaison de protéines oxydées au CD36.

13. Médicament selon l'une quelconque des revendications 1 à 12, **caractérisé en ce qu'**il contient des anticorps monoclonaux ou des fragments (F(ab)₂, F(ab), région de reconnaissance de l'antigène) de ceux-ci.

14. Médicament selon l'une quelconque des revendications précédentes, **caractérisé en ce qu'**il contient d'autres adjuvants et/ou excipients pharmaceutiquement acceptables.

15. Médicament selon l'une quelconque des revendications précédentes, **caractérisé en ce qu'**il est formulé pour une administration locale, intradermique, topique, intraveineuse, orale ou intramusculaire ou pour une administration par liposome.

16. Médicament selon l'une quelconque des revendications précédentes, **caractérisé en ce qu'**il contient d'autres principes actifs, comme par exemple des antibiotiques, des immunosuppresseurs, des partenaires d'interaction de protéines oxydées dans l'organisme.

17. Procédé de contrôle de la qualité des produits sanguins par quantification de protéines oxydées dans ces produits sanguins, **caractérisé en ce que** la quantité de protéines oxydées est déterminée à l'aide d'anticorps monoclonaux qui se lient aux protéines oxydées mais ne réagissent pas avec les protéines non oxydées.

18. Procédé selon la revendication 17, **caractérisé en ce que** les anticorps monoclonaux sont dirigés contre une parmi l'antithrombine III ox, l'albumine humaine ox ou le fibrinogène ox, ou toutes ces protéines.

19. Procédé selon la revendication 17 ou 18, **caractérisé en ce que** les anticorps monoclonaux pour la quantification sont utilisés, dans un essai ELISA ou RIA ou dans un procédé de cytométrie en flux quantitative, à la surface des cellules.
